# EUROPEAN PATENT APPLICATION

(11) **EP 2 241 333 A1**
(43) Date of publication of application: **20.10.2010**
(21) Application number: 08860018.4
(22) Date of filing: 12.12.2008
(51) Int. Cl.: A61K 45/00, A61K 31/7105, A61K 31/713, A61K 39/395, A61K 48/00, A61P 35/02, A61P 43/00

(54) **THERAPEUTIC AGENT FOR MLL LEUKEMIA AND MOZ LEUKEMIA OF WHICH MOLECULAR TARGET IS M-CSF RECEPTOR, AND USE THEREOF**

(30) Priority: 12.12.2007 JP 2007321256
(71) Applicant: National Cancer Center, Chuo-ku Tokyo 104-0045 (JP)
(72) Inventor: KITABAYASHI, Issay, Tokyo 104-0045 (JP)
(74) Representative: Plougmann & Vingtoft A/S
(86) International application number: PCT/JP2008/072609
(87) International publication number: WO 2009/075344

(57) **Abstract**

An objective of the present invention is to provide therapeutic agents for MLL leukemia and MOZ leukemia, which include an M-CSF receptor inhibitor as an active ingredient. Another objective of the present invention is to provide methods of screening for pharmaceutical compositions that treat or prevent leukemia by suppressing the expression or activity of M-CSF receptor. Still another objective of the present invention is to provide methods of testing for leukemia, which include the step of determining the expression level of an M-CSF receptor.
The present inventors conducted dedicated studies, and as a result revealed that the M-CSF receptor (M-CSFR, CSF1R, c-FMS, or CD115) is highly expressed in cells having the activity of inducing MLL or MOZ leukemia (leukemia stem cells), both of which are intractable acute leukemia. Specifically, the present inventors discovered that the M-CSF receptor signal is closely associated with the leukemia onset.

SEQUENCE LISTING

<110>  NATIONAL CANCER CENTER

<120>  Therapeutic agent for MLL leukemia and MOZ leukemia of which
molecular target is M-CSF receptor, and use thereof

<130>  KYM-A0701P

<150>  JP 2007-321256
<151>  2007-12-12

<160>  2

<170>  PatentIn version 3.4

<210>  1
<211>  3985
<212>  DNA
<213>  Homo sapiens

<400>  1
gaagggcaga cagagtgtcc aaaagcgtga gagcacgaag tgaggagaag gtggagaaga      60

gagaagagga agaggaagag gaagagagga agcggaggga actgcggcca ggctaaaagg     120

ggaagaagag gatcagccca aggaggagga agaggaaaac aagacaaaca gccagtgcag     180

aggagaggaa cgtgtgtcca gtgtcccgat ccctgcggag ctagtagctg agagctctgt     240

gccctgggca ccttgcagcc ctgcacctgc ctgccacttc cccaccgagg ccatgggccc     300

aggagttctg ctgctcctgc tggtggccac agcttggcat ggtcagggaa tcccagtgat     360

agagcccagt gtccctgagc tggtcgtgaa gccaggagca acggtgacct tgcgatgtgt     420

gggcaatggc agcgtggaat gggatggccc cccatcacct cactggaccc tgtactctga     480

tggctccagc agcatcctca gcaccaacaa cgctaccttc caaaacacgg ggacctatcg     540

ctgcactgag cctggagacc ccctgggagg cagcgccgcc atccacctct atgtcaaaga     600

ccctgcccgg ccctggaacg tgctagcaca ggaggtggtc gtgttcgagg accaggacgc     660

actactgccc tgtctgctca cagacccggt gctggaagca ggcgtctcgc tggtgcgtgt     720

gcgtggccgg cccctcatgc gccacaccaa ctactccttc tcgccctggc atggcttcac     780

catccacagg gccaagttca ttcagagcca ggactatcaa tgcagtgccc tgatgggtgg     840

caggaaggtg atgtccatca gcatccggct gaaagtgcag aaagtcatcc cagggccccc     900

agccttgaca ctggtgcctg cagagctggt gcggattcga ggggaggctg cccagatcgt 960

gtgctcagcc agcagcgttg atgttaactt tgatgtcttc ctccaacaca acaacaccaa 1020

gctcgcaatc cctcaacaat ctgactttca taataaccgt taccaaaaag tcctgaccct 1080

caacctcgat caagtagatt tccaacatgc cggcaactac tcctgcgtgg ccagcaacgt 1140

gcagggcaag cactccacct ccatgttctt ccgggtggta gagagtgcct acttgaactt 1200

gagctctgag cagaacctca tccaggaggt gaccgtgggg gaggggctca acctcaaagt 1260

catggtggag gcctacccag gcctgcaagg ttttaactgg acctacctgg gacccttttc 1320

tgaccaccag cctgagccca agcttgctaa tgctaccacc aaggacacat acaggcacac 1380

cttcaccctc tctctgcccc gcctgaagcc ctctgaggct ggccgctact ccttcctggc 1440

cagaaaccca ggaggctgga gagctctgac gtttgagctc acccttcgat accccccaga 1500

ggtaagcgtc atatggacat tcatcaacgg ctctggcacc cttttgtgtg ctgcctctgg 1560

gtacccccag cccaacgtga catggctgca gtgcagtggc cacactgata ggtgtgatga 1620

ggcccaagtg ctgcaggtct gggatgaccc ataccctgag gtcctgagcc aggagccctt 1680

ccacaaggtg acggtgcaga gcctgctgac tgttgagacc ttagagcaca accaaaccta 1740

cgagtgcagg gcccacaaca gcgtggggag tggctcctgg gccttcatac ccatctctgc 1800

aggagcccac acgcatcccc cggatgagtt cctcttcaca ccagtggtgg tcgcctgcat 1860

gtccatcatg gccttgctgc tgctgctgct cctgctgcta ttgtacaagt ataagcagaa 1920

gcccaagtac caggtccgct ggaagatcat cgagagctat gagggcaaca gttatacttt 1980

catcgacccc acgcagctgc cttacaacga gaagtgggag ttcccccgga acaacctgca 2040

gtttggtaag accctcggag ctggagcctt tgggaaggtg gtggaggcca cggcctttgg 2100

tctgggcaag gaggatgctg tcctgaaggt ggctgtgaag atgctgaagt ccacggccca 2160

tgctgatgag aaggaggccc tcatgtccga gctgaagatc atgagccacc tgggccagca 2220

cgagaacatc gtcaaccttc tgggagcctg tacccatgga ggccctgtac tggtcatcac 2280

ggagtactgt tgctatggcg acctgctcaa ctttctgcga aggaaggctg aggccatgct 2340

gggacccagc ctgagccccg gccaggaccc cgagggaggc gtcgactata agaacatcca 2400

cctcgagaag aaatatgtcc gcagggacag tggcttctcc agccagggtg tggacaccta 2460

tgtggagatg aggcctgtct ccacttcttc aaatgactcc ttctctgagc aagacctgga 2520

caaggaggat ggacggcccc tggagctccg ggacctgctt cacttctcca gccaagtagc 2580

ccagggcatg gccttcctcg cttccaagaa ttgcatccac cgggacgtgg cagcgcgtaa 2640

cgtgctgttg accaatggtc atgtggccaa gattggggac ttcgggctgg ctagggacat 2700

catgaatgac tccaactaca ttgtcaaggg caatgcccgc ctgcctgtga agtggatggc 2760

cccagagagc atctttgact gtgtctacac ggttcagagc gacgtctggt cctatggcat 2820

cctcctctgg gagatcttct cacttgggct gaatccctac cctggcatcc tggtgaacag 2880

caagttctat aaactggtga aggatggata ccaaatggcc cagcctgcat ttgccccaaa 2940

gaatatatac agcatcatgc aggcctgctg ggccttggag cccacccaca gacccacctt 3000

ccagcagatc tgctccttcc ttcaggagca ggcccaagag gacaggagag agcgggacta 3060

taccaatctg ccgagcagca gcagaagcgg tggcagcggc agcagcagca gtgagctgga 3120

ggaggagagc tctagtgagc acctgacctg ctgcgagcaa ggggatatcg cccagccctt 3180

gctgcagccc aacaactatc agttctgctg aggagttgac gacagggagt accactctcc 3240

cctcctccaa acttcaactc ctccatggat ggggcgacac ggggagaaca tacaaactct 3300

gccttcggtc atttcactca acagctcggc ccagctctga aacttgggaa ggtgagggat 3360

tcaggggagg tcagaggatc ccacttcctg agcatgggcc atcactgcca gtcaggggct 3420

gggggctgag ccctcacccc cccctcccct actgttctca tggtgttggc ctcgtgtttg 3480

ctatgccaac tagtagaacc ttctttccta atccccttat cttcatggaa atggactgac 3540

tttatgccta tgaagtcccc aggagctaca ctgatactga gaaaaccagg ctctttgggg 3600

ctagacagac tggcagagag tgagatctcc ctctctgaga ggagcagcag atgctcacag 3660

accacactca gctcaggccc cttggagcag gatggctcct ctaagaatct cacaggacct 3720

cttagtctct gccctatacg ccgccttcac tccacagcct cacccctccc acccccatac 3780

tggtactgct gtaatgagcc aagtggcagc taaaagttgg gggtgttctg cccagtcccg 3840

tcattctggg ctagaaggca ggggaccttg gcatgtggct ggccacacca agcaggaagc 3900

acaaactccc ccaagctgac tcatcctaac taacagtcac gccgtgggat gtctctgtcc 3960

acattaaact aacagcatta atgca 3985

<210>  2
<211>  972
<212>  PRT
<213>  Homo sapiens

<400>  2

## Description

### Technical Field

The present invention relates to therapeutic agents for MLL leukemia and MOZ leukemia, which comprise as an active ingredient a macrophage colony stimulating factor (M-CSF) receptor inhibitor, and uses thereof. The present invention also relates to methods of screening for pharmaceutical compositions for treating or preventing leukemia by suppressing expression or activity of M-CSF receptor. Furthermore, the present invention relates to methods of testing for leukemia, which comprise the step of determining the expression level of M-CSF receptor.

### Background Art

Leukemia, which is a blood cancer, is classified into chronic myelogenous leukemia and acute leukemia based on the nature of the leukemia cells. Acute leukemia is subdivided into acute myelogenous leukemia and acute lymphocytic leukemia. It has been demonstrated that specific chromosomal translocation is frequently observed in human leukemia and the expression of the resulting fusion protein is crucial for leukemia onset (Non-patent Document 1). MLL gene has been reported to frequently serve as a target of chromosomal translocation in acute myelogenous leukemia and acute lymphocytic leukemia, and to fuse with 40 or more various genes (Non-patent Documents 2 and 3). MOZ gene has been reported to serve as a target of some chromosomal translocation found in acute myelogenous leukemia, and to fuse with genes such as CBP, p300, or TIF2 (Non-patent Documents 4 to 8).

The t(9;22) translocation is often observed in chronic myelogenous leukemia. The expression of the resulting Breakpoint Cluster Region (BCR)-abelson strain of murine leukemia virus (ABL) fusion protein is closely associated with leukemia onset (Non-patent Documents 9 and 10). Imatinib (Glivec; Novartis Pharma, Switzerland) whose target is this BCR-ABL was recently revealed to be effective for chronic myelogenous leukemia, and is thus widely used (Non-patent Document 11). Meanwhile, the current primary therapy for acute leukemia is chemotherapy. However, leukemia is often recurrent after chemotherapy-induced remission, and the recurrence is closely associated with the survival rate. All-trans retinoic acid is effective against some acute leukemia (acute promyelocytic leukemia); however, effective molecular-targeted therapeutic agents still remain unavailable for the majority of other patients. In particular, development of effective therapeutic methods for MLL leukemia (leukemia with the MLL fusion gene expression) and MOZ leukemia (leukemia with the MOZ fusion gene expression) has been needed, because these leukemia have poor prognosis.

Prior art document information relevant to the invention of the present application is shown below.
[Non-patent Document 1] Look, A.T., Science (1997) 278, 1059-1064
[Non-patent Document 2] Huret, J. L., et al., Leukemia (2001) 15, 987-989
[Non-patent Document 3] Meyer, C., et al., Leukemia (2006) 20, 777-784
[Non-patent Document 4] Borrow, J., et al., Nat. Genet. (1996) 14, 33-41
[Non-patent Document 5] Carapeti, M., et al., Blood (1998) 91, 3127-3133
[Non-patent Document 6] Liang, J., et al., Blood (1998) 92, 2118-2122
[Non-patent Document 7] Chaffanet, M., et al., Genes Chromosomes Cancer (2000) 28, 138-144
[Non-patent Document 8] Kitabayashi, I., et al., Leukemia (2001) 15, 89-94
[Non-patent Document 9] Faderl, S. et al., N Engl J Med (1999) 341, 164-172
[Non-patent Document 10] Sawyers, CL., et al., N Engl J Med (1999) 340, 1330-1340
[Non-patent Document 11] Druker, BJ., et al., Nat. Med. (1996) 2, 561-566

### [Disclosure of the Invention]

### [Problems to be Solved by the Invention]

The present invention was achieved in view of the circumstances described above. An objective of the present invention is to provide therapeutic agents for MLL leukemia and MOZ leukemia, which comprise an M-CSF receptor inhibitor as an active ingredient. Another objective of the present invention is to provide methods of screening for pharmaceutical compositions for treating or preventing leukemia by suppressing an expression or activity of M-CSF receptor. Still another objective of the present invention is to provide methods of testing for leukemia, which comprise the step of determining the expression level of the M-CSF receptor.

### [Means for Solving the Problems]

To achieve the above-described objectives, the present inventors studied the role of M-CSF receptor in MLL leukemia (leukemia with the MLL fusion gene expression) and MOZ leukemia (leukemia with the MOZ fusion gene expression), both of which are intractable acute leukemia.

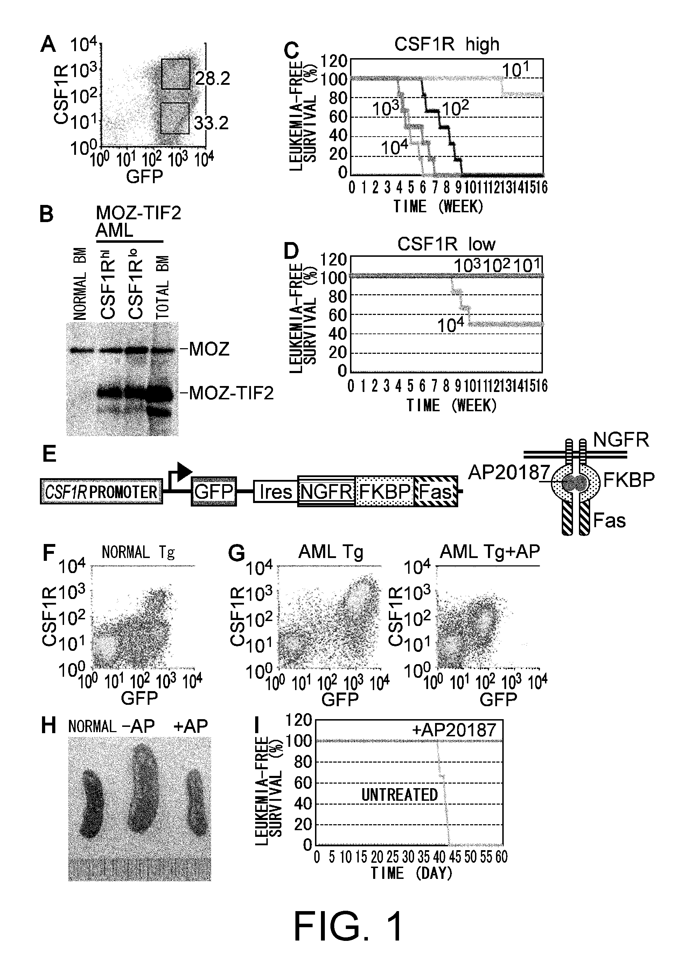

First, the present inventors determined the expression level of M-CSF receptor in cells having the activity of inducing MLL leukemia or MOZ leukemia (leukemia stem cells). The result showed that there were two types of cells: M-CSFR^{high} cells and M-CSFR^{low/-} cells expressing M-CSFR (abbreviated as CSF1R in the Drawings) at high and low levels, respectively (Fig. 1A), while both M-CSFR^{hign} and M-CSFR^{low/-} cells express MOZ-TIF2 protein at equivalent levels (Fig. 1B).

Then, the present inventors sorted M-CSFR^{high} cells and M-CSFR^{low/-} cells using a cell sorter, transplanted them into mice, and assayed their leukemia-inducing activity. The result showed that M-CSFR^{high} cells had a strong leukemia-inducing activity while M-CSFR^{low/-} cells had low leukemia-inducing activity (Figs. 1C and 1D).

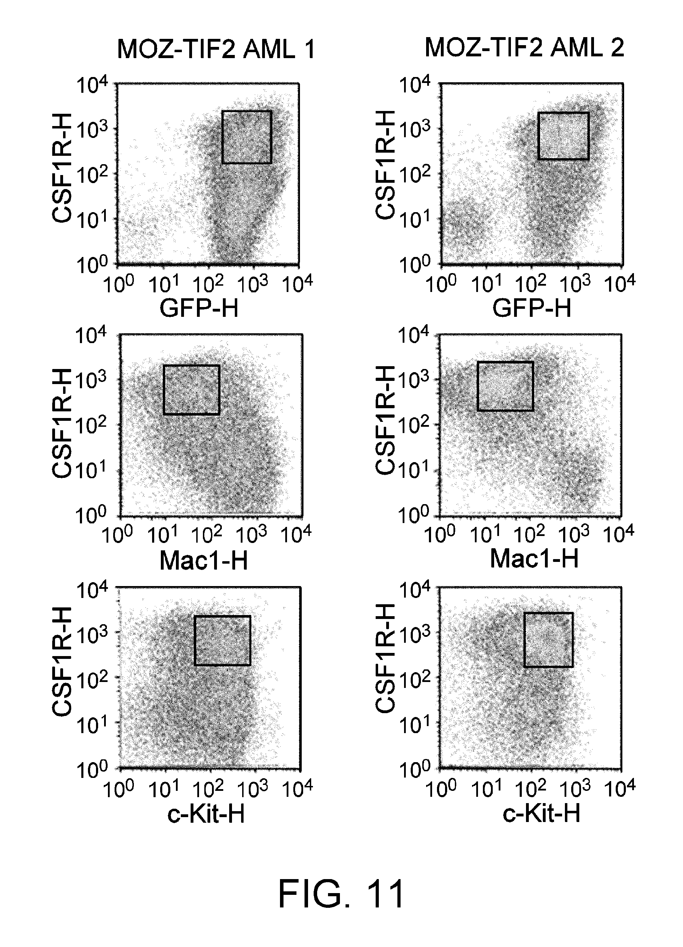

Next, whether leukemia onset was suppressed by removing M-CSFR^{high} cells that were assumed to include LIC was assessed by testing whether AML is developed in transgenic mice expressing EGFP and a drug inducible FKBP-Fas suicide gene. According to the result, an increase in the M-CSFR^{high} cell count (Fig. 1G) and splenomegaly (Fig. 1H) were confirmed in untreated mice, implying leukemia onset (Fig. 11), while both splenomegaly (Fig. 1H) and leukemia onset (Fig. 1I) were suppressed in mice administered with an AP20187 dimerizer to eliminate cells expressing M-CSFR (Fig. 1G). This result demonstrates that leukemia onset can be suppressed by eliminating cells expressing M-CSFR, suggesting that M-CSFR-targeted therapy is effective.

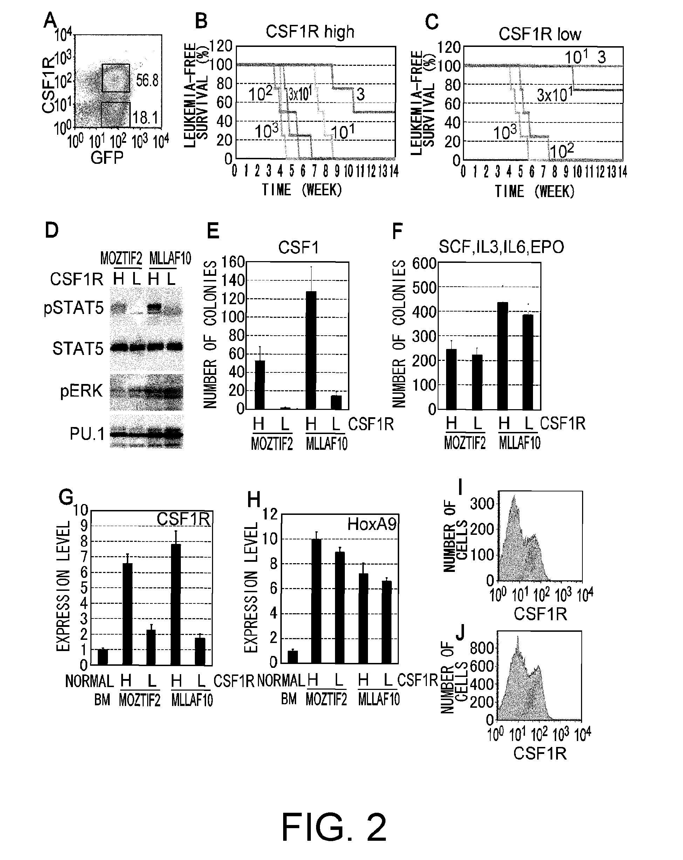

The present inventors also discovered that M-CSFR^{high} cells and M-CSFR^{low/-} cells were present in the bone narrow of MLL-AF 10-induced AML mice (Fig. 2A) and M-CSFR^{high} cells had strong LIC activity (Fig. 2B). Furthermore, the present inventors revealed that STAT5, a downstream effector of M-CSFR, was specifically activated in M-CSFR^{high} cells of MOZ-TIF2-induced AML mice and MLL-AF10-induced AML mice (Fig. 2D).

Next, the present inventors isolated M-CSFR^{high} cells and M-CSFR^{low/-} cells using a cell sorter and tested for a colony-forming ability of the cells in the presence of either M-CSF/CSF1 or several types of cytokines. The result showed that, in the presence of CSF1, only M-CSFR^{high} cells formed colonies (Fig. 2E), while in the presence of several types of cytokines, M-CSFR^{high} cells and M-CSFR^{low/-} cells formed the same number of colonies (Fig. 2F). This suggests that functional M-CSFR is expressed in M-CSFR^{high} cells.

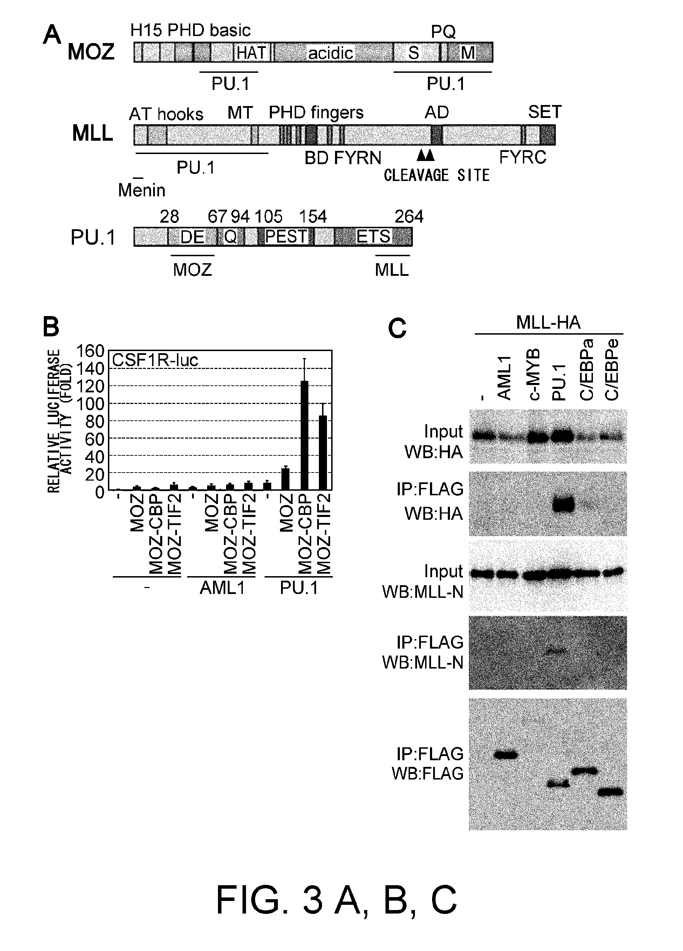

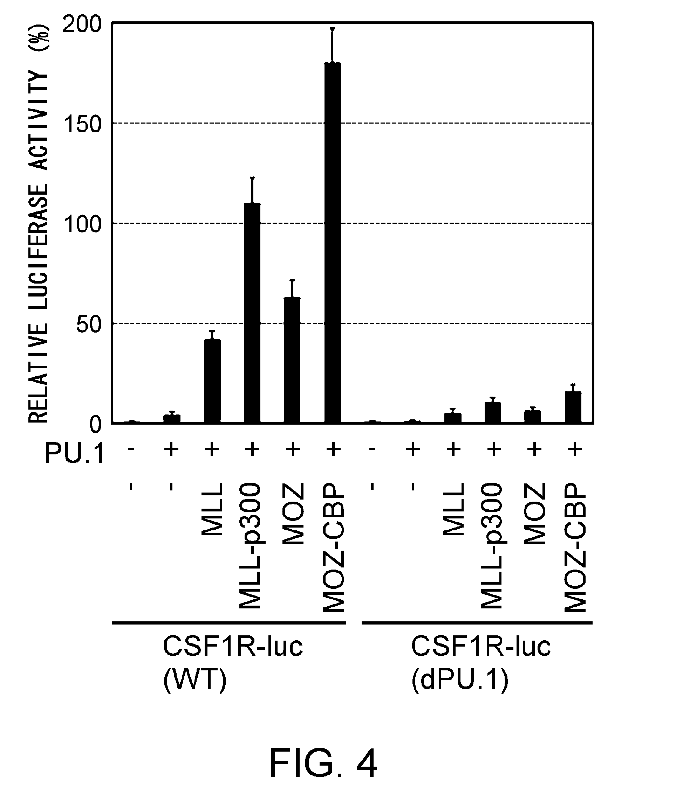

Then, the regulation of M-CSFR expression by the MOZ fusion gene or MLL fusion gene was assessed using a reporter gene in which the luciferase gene is linked to a transcriptional regulatory region of M-CSFR. The result showed that the transcription of M-CSFR was activated by MOZ, MLL, and their fusion proteins in a PU.1-dependent manner (Figs. 3B to 3D, and 4).

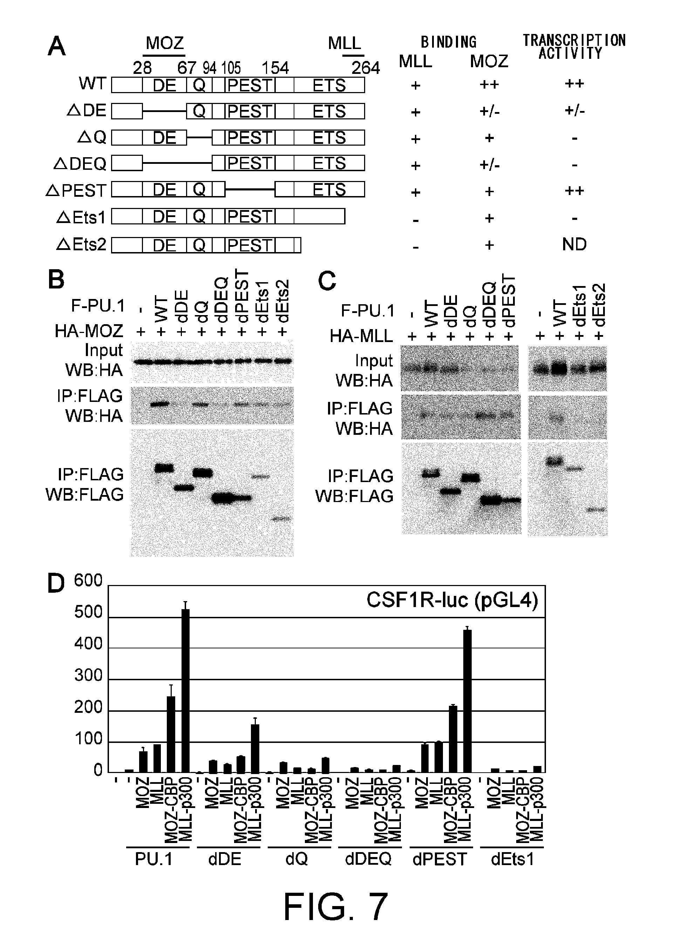

Furthermore, an analysis using deletion mutants revealed that the acidic amino acid-rich region (DE region) or ETS domain in PU.1 is required for the binding between PU.1 and MOZ or MLL, respectively (Figs. 7B and 7C). Both domains were demonstrated to be essential for the PU.1-meidated transcriptional activation by MOZ, MLL, MOZ-CBP, and MLL-p300 (Fig. 7D).

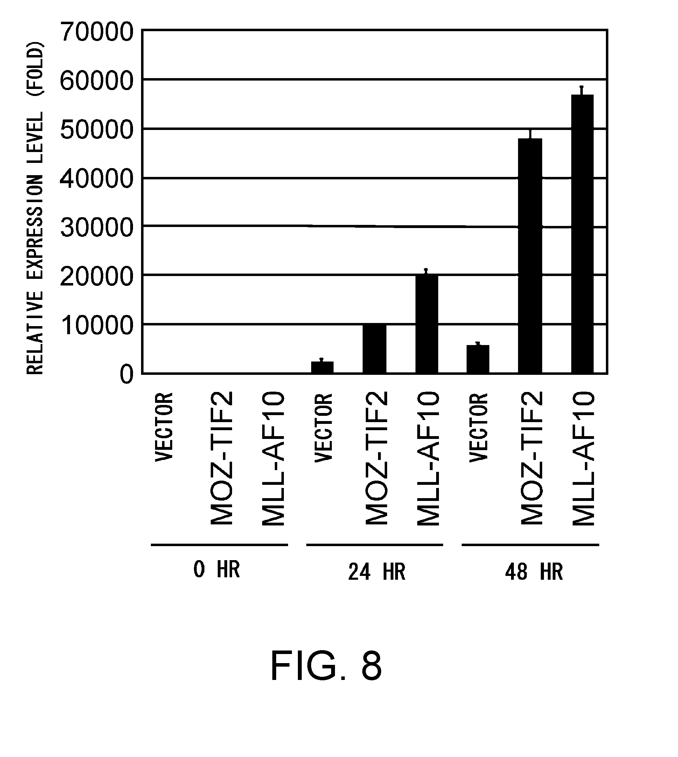

Furthermore, MOZ-TIF2 or MLL-AF10 was expressed in PU.1-/- (PU.1-ER) cells and M-CSFR expression in the cells was examined using a cell sorter. The result showed that M-CSFR expression was increased when PU.1-ER was activated by adding 4-hydroxytamoxifen, and the expression of MOZ-TIF2 and MLL-AF10 significantly enhanced the increased expression (Fig. 8).

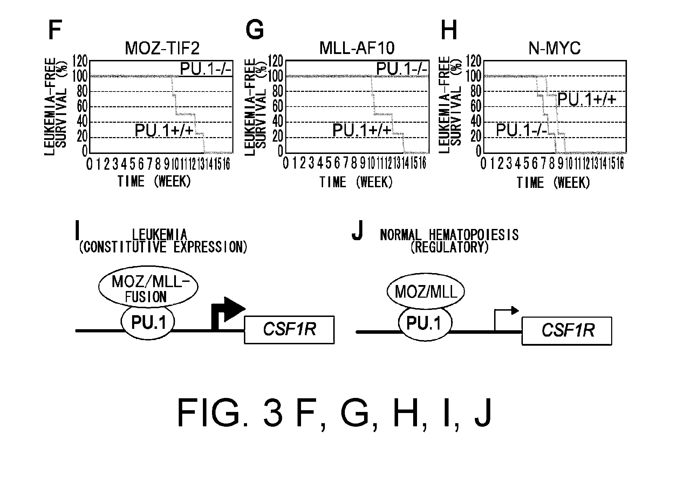

Next, MOZ-TIF2 and MLL-AF10 were expressed in fetal hepatocytes from PU.1-deficient mice and the resulting cells were transplanted into mice to test whether leukemia is developed in the mice. The result showed that PU.1 was essential for MOZ-TIF2- and MLL-AF10-induced leukemia (Figs. 3F-I).

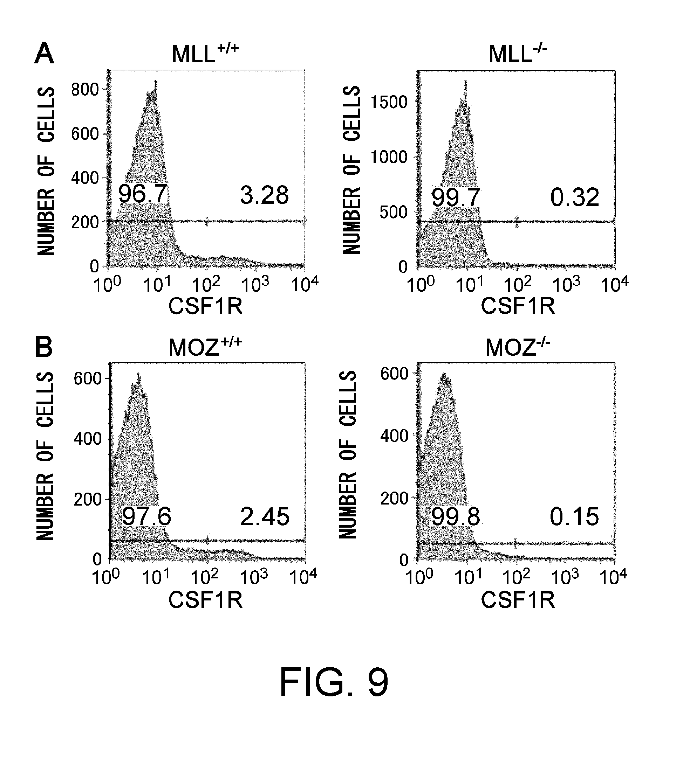

Furthermore, it was demonstrated that M-CSFR expression was impaired in MOZ-/- or MLL-/- fetal hepatocytes (Fig. 9A and 9B).

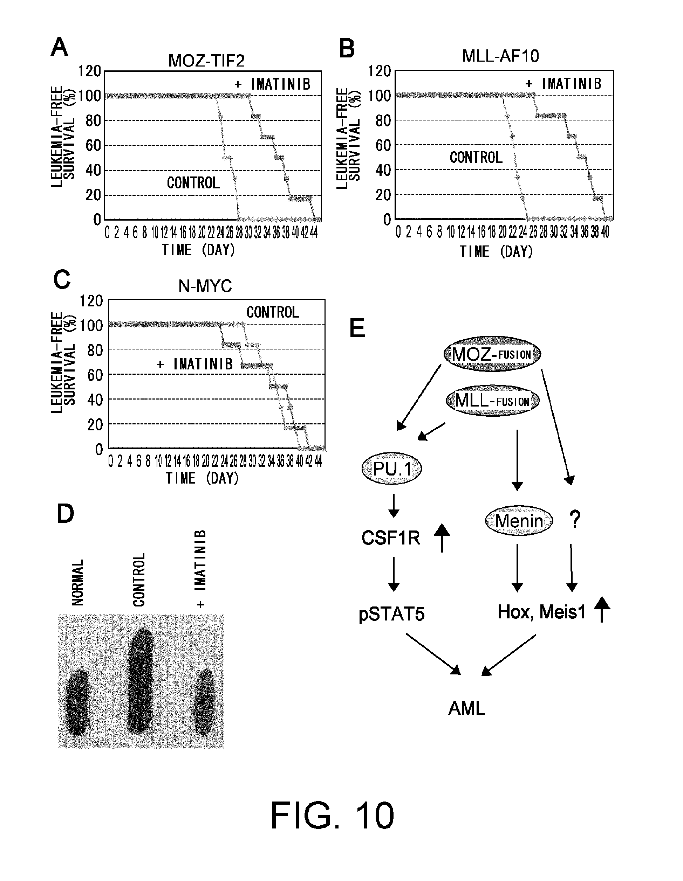

Then, bone marrow cells from mice with leukemia induced by the MOZ-TIF2 fusion gene and MLL-AF10 gene were transplanted into irradiated wild-type mice, and seven days after transplantation Imatinib mesylate (STI571; Glivec, Gleevec^{®}) was administered to them to assess its therapeutic effect against leukemia. The result showed that Imatinib, an M-CSF receptor inhibitor, had an effect of delaying the leukemia onset in the leukemia model mouse (Figs. 10A-D).

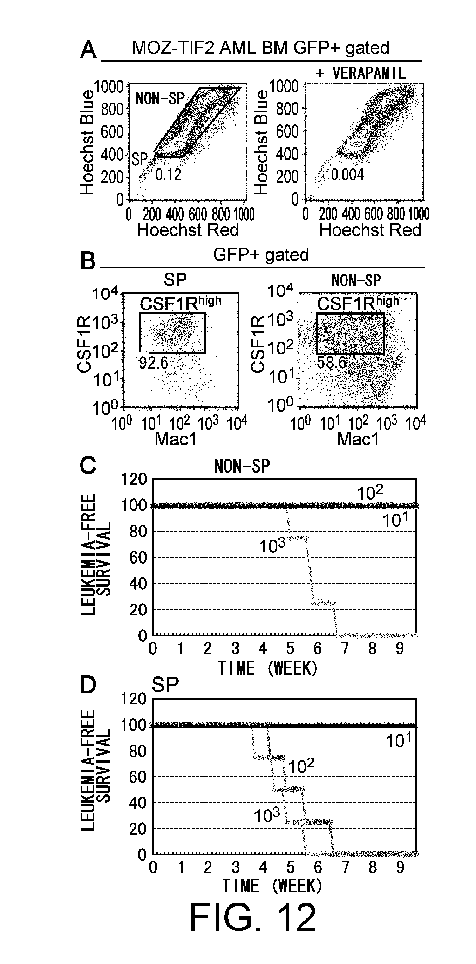

In addition, the present inventors revealed that M-CSFR^{high}-LIC was c-Kit⁺ Sca-1⁻CD16/32⁺ Mac-1^{low} Gr-1⁺ (Fig. 11) and side population (SP) cells, characteristic of some normal and malignant stem cells, were present in the bone marrow of MOZ-TIF2-induced AML mice (Figs. 12A-D). The present inventors also demonstrated that MLL fusion and MOZ fusion induced increased expression of receptor tyrosine kinase M-CSFR by interacting with PU.1. This activated two types of pathways, resulting in early onset of AML (Fig. 10E).

Specifically, the present inventors demonstrated that M-CSF receptor (M-CSFR, CSF1R, c-FMS, or CD115) was expressed at a high level in cells having the activity of inducing MLL leukemia and MOZ leukemia (leukemia stem cells), both of which are intractable acute leukemia, implying that M-CSF receptor signal is closely associated with leukemia onset. Thus, the present inventors completed the present invention.

### More specifically, the present invention provides the following [1] to [17]:

[1] a therapeutic agent for MLL leukemia or MOZ leukemia comprising an M-CSF receptor inhibitor as an active ingredient;
[2] the therapeutic agent for leukemia of [1], wherein the M-CSF receptor inhibitor is an agent that inhibits tyrosine kinase activity of the M-CSF receptor;
[3] the therapeutic agent of [1] for leukemia, wherein the M-CSF receptor inhibitor is an anti-M-CSF receptor antibody;
[4] the therapeutic agent for leukemia of [1], wherein the M-CSF receptor inhibitor is an aptamer against the M-CSF receptor;
[5] the therapeutic agent for leukemia of [1], wherein the M-CSF receptor inhibitor is an antisense RNA or dsRNA;
[6] the therapeutic agent for leukemia of [1], wherein the M-CSF receptor inhibitor is Imatinib mesylate;
[7] the therapeutic agent for leukemia of any one of [1] to [6], wherein the M-CSF receptor is any one of M-CSFR, CSF1R, c-FMS, or CD115;
[8] a method of treating or preventing leukemia in a subject, which comprises the step of administering an M-CSF receptor inhibitor to the subject;
[9] use of an M-CSF receptor inhibitor in the manufacture of a therapeutic agent for leukemia;
[10] a method of screening for a pharmaceutical composition that treats or prevents leukemia by suppressing an expression of an M-CSF receptor, which comprises the steps of:
   (a) contacting a test compound with an M-CSF receptor;
   (b) detecting the binding of a test compound to the M-CSF receptor; and
   (c) selecting a test compound that binds to the M-CSF receptor;
[11] a method of screening for a pharmaceutical composition that treats or prevents leukemia by suppressing the expression of an M-CSF receptor, which comprises the steps of:
   (a) contacting a test compound with a cell expressing an M-CSF receptor gene;
   (b) determining the expression level of the M-CSF receptor; and
   (c) selecting a test compound that reduces the expression level of the M-CSF receptor as compared to when the test compound is not contacted;
[12] a method of screening for a pharmaceutical composition that treats or prevents leukemia by suppressing the expression of an M-CSF receptor, which comprises the steps of:
   (a) providing a cell or cell extract comprising a DNA wherein a reporter gene is operably linked to downstream of the promoter region of DNA encoding an M-CSF receptor;
   (b) contacting a test compound with the cell or cell extract;
   (c) determining the expression level of the reporter gene in the cell or cell extract; and
   (d) selecting a test compound that reduces the expression level of the reporter gene as compared to when the test compound is not contacted;
[13] a method of screening for a pharmaceutical composition that treats or prevents leukemia by suppressing the activity of an M-CSF receptor, which comprises the steps of:
   (a) contacting a test compound with a cell expressing an M-CSF receptor;
   (b) determining the activity of the M-CSF receptor in the cell; and
   (c) selecting a test compound that reduces the activity of the reporter gene as compared to when the test compound is not contacted;
[14] the method of any one of [10] to [13] of screening for a pharmaceutical composition, wherein the leukemia is MLL or MOZ leukemia;
[15] a method of testing for leukemia, which comprises the step of determining the expression level of an M-CSF receptor;
[16] an agent for diagnosing leukemia, which is used in the test method of [15], and which comprises an oligonucleotide with a length of at least 15 nucleotides and which hybridizes to a region of the M-CSF receptor gene; and
[17] the agent for diagnosing leukemia, which is used in the test method of [15], and which comprises an antibody that recognizes an M-CSF receptor.

### [Brief Description of the Drawings]

Fig. 1 depicts the result of M-CSFR expression in bone marrow cells of MOZ leukemogenesis mice. (A) BM cells from MOZ-TIF2-induced AML mice were analyzed by FACS for expression of GFP and M-CSFR. (B) M-CSFR^{high} cells and M-CSFR^{low/-} cells were sorted by flow cytometer. MOZ-TIF2 expression in these cells was investigated by immunoblot analysis using an anti-MOZ antibody. (C, D) Indicated numbers of M-CSFR^{high} cells (C) and M-CSFR^{low/-} cells (D) were transplanted into mice irradiated beforehand with a lethal dose of radiation and survival from leukemia onset was investigated. (E) Structure of genes for the *M-CSFR* promoter, EGFP, the NGFR-FKBP-Fas suicide construct, and activation mechanism of NGFR-FKBP-Fas. In transgenic (Tg) mice, conditional ablation of cells highly expressing M-CSFR can be induced by exposure to an AP20187 dimerizer. (F-I) AML mice were induced by transplantation of BM cells from Tg mice infected with MOZ-TIF2, and 10⁵ BM cells from AML Tg mice were secondary transplanted into wild-type mice lethally-irradiated beforehand. The secondary-transplanted mice were exposed to AP20187 seven days after transplantation. Expression of GFP and M-CSFR in BM cells (F, G) and the size of spleens (H) of normal Tg mice (F), and of untreated and AP20187-treated secondary-transplanted mice (G) were analyzed four weeks after transplantation. Leukemia-free survival rate of the untreated and AP20187-treated secondary-transplanted mice were investigated (I).
Fig. 2 shows the result of determining HoxA9 expression, STAT5 phosphorylation, and the colony-forming activity of M-CSFR^{high} and M-CSFR^{low/-} cells. (A) Bone marrow cells from MLL-AF10-induced AML mice were analyzed by FACS for expression of GFP and M-CSFR. (B, C) Indicated numbers of cell sorter-sorted M-CSFR^{high} cells (B) and M-CSFR^{low/-} cells (C) were transplanted into wild-type mouse lethally-irradiated beforehand and leukemia-free survival was investigated. (D) M-CSFR^{high} cells and M-CSFR^{low/-} cells were analyzed by immunoblotting for expression of phosphorylated STAT5 (pSTAT5), total STAT5, phosphorylated ERK (pERK), and PU.1. (E, F) M-CSFR^{high} cells and M-CSFR^{low/-} cells were tested for their colony-forming abilities on methylcellulose medium with M-CSF alone (E), or with SCF, IL3, IL6, and erythropoietin (F). (G, H) Expression levels of M-CSFR (G) and HoxA9 (H) in M-CSFR^{high}, M-CSFR^{low/-}, and normal BM cells were analyzed by quantitative RT-PCR. (I, J) Bone marrow cells from an AML patient with t(8;16) (I) and THP-1 cells with t(9;11) (J), which were cultured for three days in 10 ng/ml hM-CSF, were tested by FACS for M-CSFR expression.
Fig. 3 (A) shows protein-interacting domains on MOZ, MLL, and PU.1. (B) shows the effects of MOZ, MOZ-CBP, and MOZ-TIF2 on AML1- and PU.1-mediated transcription of the M-CSFR promoter. SaOS2 cells were transfected with M-CSFR-luciferase and the effectors indicated. Luciferase activity was analyzed 24 h after transfection. (C) shows an interaction of MLL with PU.1. Photograph shows the result of transfecting MLL-HA into 293T cells together with either FLAG-tagged AML1, c-MYB, PU.1, C/EBPa, or C/EBPe. Immunoprecipitates with anti-FLAG antibody (IP: FLAG) or cell lysates (Input) were immunoblotted with anti-HA, anti-MLL (N-terminus), or anti-FLAG antibodies. (D) shows the effects of MLL and MLL-fusions on PU.1-mediated transcription of M-CSFR promoter. (E) shows the result of M-CSFR expression analysis by FACS using PUER cells infected with MSCV-GFP, MSCV-MOZ-TIF2-ires-GFP, or MSCV-MLL-AF 10-ires-GFP retroviruses and exposed to 100 nM 4-Hydroxytamoxifen (4-HT) for two to five days. (F-H) show the results of analyzing leukemia-free survival of mice. Fetal liver cells of E12.5 PU.1^{+/+} and PU.1^{-/-} mouse embryo littermates were infected with either MOZ-TIF2 (F), MLL-AF10 (G), or N-MYC (H), and transplanted into irradiated mice. (I) and (J) show models for transcriptional regulation by normal and fusion MOZ/MLL proteins. MOZ- and MLL-fusions stimulated constitutive expression of M-CSFR to induce leukemia (I). Normal MOZ and MLL control M-CSFR expression by binding to PU.1 to regulate normal hematopoiesis (J).
Fig. 4 shows the effects of MOZ, MLL, MOZ-CBP, and MLL-p300 on PU.1-mediated transcription of the *CSF1R* promoter. SaOS2 cells were transfected with wild-type *CSF1R*-luciferase or its mutant lacking the PU.1-binding site, together with effectors indicated. Luciferase activity was analyzed 24 h after transfection.

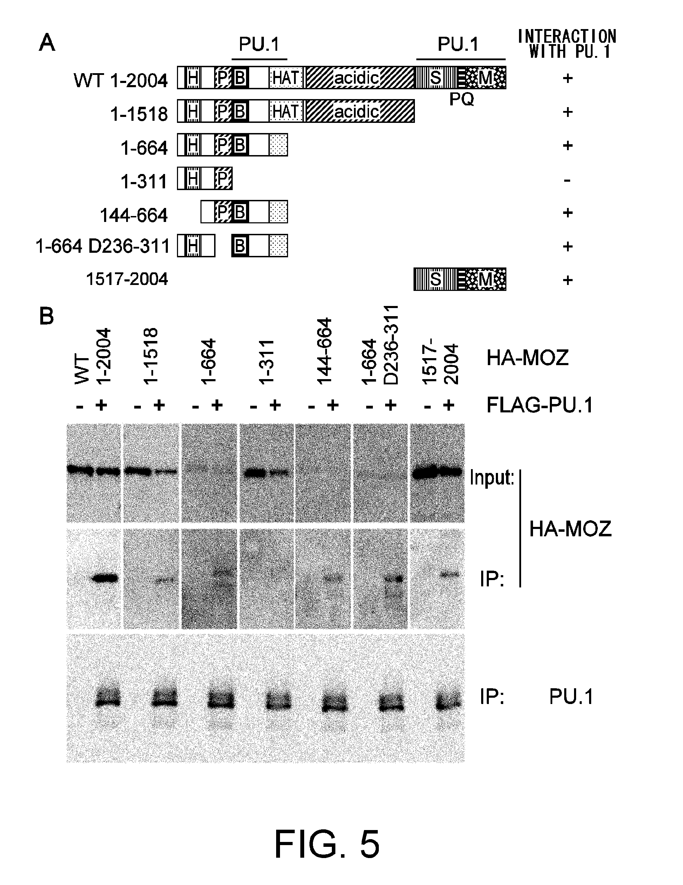

Fig. 5 depicts a figure and photograph showing the interaction of MOZ mutants with PU.1. (A) shows the structure of MOZ deletion mutants. (B) depicts a photograph showing the result of transfecting wild-type and mutants of HA-tagged MOZ into 293T cells together with FLAG-tagged PU.1. Immunoprecipitates with anti-FLAG antibody (IP: FLAG) or cell lysates (Input) were immunoblotted with anti-HA or anti-FLAG antibodies.

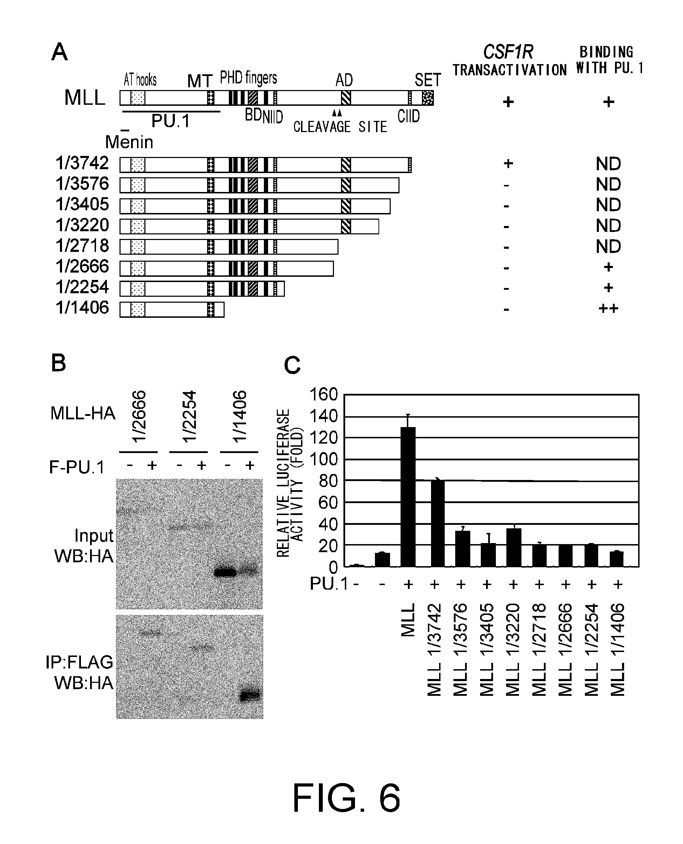

Fig. 6. (A) shows the structure of MLL deletion mutants. (B) depicts a photograph showing an interaction of PU.1 with MLL deletion mutants. 293T cells were transfected with deletion mutants of HA-tagged MLL together with FLAG-tagged PU.1. Immunoprecipitates with anti-FLAG antibody (IP: FLAG) or cell lysates (Input) were immunoblotted with anti-HA or anti-FLAG antibodies. (C) shows the effect of MLL mutants on PU.1-mediated transcription of *CSF1R* promoter. SaOS2 cells were transfected with CSF1R-luciferase together with PU.1 and MLL mutants. Luciferase activity was analyzed 24 h after transfection.
Fig. 7. (A) shows the structure of PU.1 deletion mutants. (B, C) depict photographs showing interactions of PU.1 mutants with MOZ (B) and MLL (C). 293T cells were transfected with HA-tagged MOZ (B) or MLL (C) together with wild-type and mutants of FLAG-tagged PU.1. Immunoprecipitates with anti-FLAG antibody (IP: FLAG) or cell lysates (Input) were immunoblotted with anti-HA or anti-FLAG antibodies. (D) shows the effects of MOZ or MLL on activity of PU.1 mutants. SaOS2 cells were transfected with CSF1R-luciferase together with wild-type or mutants of PU.1, MOZ, MLL, MOZ-CBP, or MLL-p300. Luciferase activity was analyzed 24 h after transfection.
Fig. 8 depicts the result showing the CSF1R mRNA expression of PUER cells infected with MSCV-GFP, MSCV-MOZ-TIF2-ires-GFP, or MSCV-MLL-AF10-ires-GFP retroviruses by exposing to 100 nM 4-Hydroxytamoxifen (4-HT) for 24 or 48 h and analyzing by quantitative RT-PCR.
Fig. 9 shows the result of analyzing expressions of CSF1R in fetal liver cells of E12.5 MLL^{+/+} and MLL^{-/-} mice embryo littermates (A) and E14.5 MOZ^{+/+} and MOZ^{-/-} mice embryo littermates (B).
Fig. 10 (A-D) BM cells (10⁵ cells) from AML mice with MOZ-TIF2 (A), MLL-AF10 (B), or N-MYC (C) were transplanted into irradiated mice. Imatinib mesylate was administrated twice a day. (A-C) show the results of analyzing leukemia-free survival of the mice. (D) depicts the photograph showing the result of analyzing spleen sizes of the MOZ-TIF2-introduced mice three weeks after transplantation. (E) shows model for the pathogenesis of AML induced by MLL- and MOZ-fusion proteins. MOZ- and MLL-fusions upregulate Hox and *Meisl* genes as well as *M-CSFR* to induce AML.
Fig. 11 shows the result of analyzing GFP, CSF1R, Mac-1 and c-Kit expression of BM cells from MOZ-TIF2 induced AML mice by FACS.
Fig. 12 (A) shows the result of analyzing SP cells by staining bone marrow cells from MOZ-TIF2-induced AML mice with Hoechst 33342 in the presence or absence of verapamil. (B) Bone marrow cells from the AML mice were stained with anti-CSF1R-PE, anti-Macl-PE-Cy7, and Hoechst 33342. GFP⁺ SP cells (right panel) and GFP⁺ non-SP cells were analyzed for expression of CSF1R and Mac1. (C, D) Indicated numbers of flow-sorted non-SP cells (C) and SP cells (D) were transplanted into lethally-irradiated mice in advance and leukemia-free survival were investigated.

### Mode for Carrying Out the Invention

The present inventors found that M-CSF receptor (M-CSFR, CSF1R, c-FMS, or CD115) was highly expressed in cells having the activity of inducing MLL leukemia or MOZ leukemia (leukemia stem cells), both of which is intractable acute leukemia. The present invention is based on this finding.

The present invention relates to therapeutic agents for MLL leukemia and MOZ leukemia, which comprise an M-CSF receptor inhibitor as an active ingredient.

Herein, the "M-CSF receptor inhibitor" may be a substance that inhibits the expression or activity of M-CSF receptor. The M-CSF receptor inhibitor is preferably a substance that inhibits the tyrosine kinase activity of M-CSF receptor, or a substance having the activity of inhibiting the binding of M-CSF to M-CSF receptor. Herein, the "M-CSF receptor" includes M-CSFR, CSF1R, c-FMS, and CD115. The inhibitor of the present invention may be an inhibitor that inhibits the expression or activity of any one of proteins constituting the complex.

The M-CSF receptor inhibitor of the present invention includes, for example, tyrosine kinase activity inhibitors, anti-M-CSF receptor antibodies, anti-M-CSF antibodies, or aptamers, antisense RNAs, dsRNAs or ribozymes against the M-CSF receptor. The M-CSF receptor inhibitor of the present invention also includes, but is not particularly limited to, modified M-CSF, soluble modified M-CSF receptor, dominant-negative forms such as partial peptides of M-CSF receptor or M-CSF, and low molecular weight substances exhibiting a similar M-CSF receptor-inhibiting activity as that of the above substances. The tyrosine kinase activity inhibitors include, for example, Imatinib mesylate (Gleevec^{®}, Novartis Pharma, Switzerland). The M-CSF receptor inhibitors of the present invention include the tyrosine kinase activity inhibitors, and preferably include anti-M-CSF receptor antibodies, or aptamers, antisense RNAs, or dsRNAs against the M-CSF receptor.

Anti-M-CSF receptor antibodies or anti-M-CSF antibodies to be used in the present invention can be obtained as polyclonal or monoclonal antibodies by using known methods. The origin of the antibodies is not particularly restricted in the present invention; however, the antibodies are preferably derived from mammals, and more preferably derived from humans. The monoclonal antibodies derived from mammals include those produced by hybridomas and those produced by hosts transformed with an expression vector that contains an antibody gene using genetic engineering methods. By binding to M-CSF receptor or M-CSF, the antibodies inhibit the binding of M-CSF to M-CSF receptor, and thus block the transmission of the biological activity of M-CSF receptor into the cell (M-CSF receptor signal). Furthermore, the antibodies bind to M-CSF receptor expressed on target cells, and Fc domains of the antibodies bind to an Fc receptor on effector cells such as natural killer cells (NK cells) and macrophages. This results in transduction of cytolytic signal into the target cells, leading to death of the cell (antibody-dependent cellular cytotoxicity (ADCC)).

Basically, antibody-producing hybridomas can be prepared as follows, using conventional techniques. Specifically, such hybridomas can be prepared by using M-CSF receptor or M-CSF as a sensitizing antigen to carry out immunizations according to conventional immunization methods, fusing the resulting immunocytes with known parent cells according to conventional cell fusion methods, and screening for monoclonal antibody-producing cells according to conventional screening methods.

More specifically, anti-M-CSF receptor antibodies can be prepared as follows. For example, human M-CSF receptor and human M-CSF to be used as the sensitizing antigen for preparing antibodies can be obtained using the M-CSF receptor gene/amino acid sequence disclosed in known references (GenBank accession No. NM_005211). The nucleotide sequence of M-CSF receptor cDNA derived from human used in the methods of the present invention is shown in SEQ ID NO: 1, and the amino acid sequence of M-CSF receptor encoded by the DNA is shown in SEQ ID NO: 2.

Any M-CSF receptor may be employed as the M-CSF receptor protein, as long as it can be used as a sensitizing antigen for preparing the anti-M-CSF receptor antibodies utilized in the present invention. Herein, the "M-CSF receptor" refers to the receptor complex or the respective components (proteins) of the complex.

The genetic sequence of M-CSF receptor or M-CSF is inserted into a known expression vector system, and appropriate host cells are transformed with the vector. Then, the protein of interest is purified by known methods from the host cells or from its culture supernatant. The purified protein may be used as the sensitizing antigen. Alternatively, chemically-synthesized M-CSF receptor or M-CSF protein may be used as the sensitizing antigen. Alternatively, it is possible to use fusion proteins between M-CSF receptor or M-CSF protein and other proteins as the sensitizing antigen.

Mammals that are immunized with a sensitizing antigen are not particularly limited, though it is preferable to take into consideration compatibility with the parent cells used for cell fusion. Thus, rodents such as mice, rats, or hamsters are generally selected.

Immunization of animals with a sensitizing antigen is performed according to known methods. For example, standard methods of delivering sensitizing antigen to mammals involve intraperitoneal or subcutaneous injection. More specifically, an appropriate amount of sensitizing antigen may be diluted and suspended with PBS (phosphate-buffered saline), physiological saline, or such. If desired, this may be mixed with an appropriate amount of a standard adjuvant, such as Freund's complete adjuvant, made into an emulsion, and then preferably administered to mammals several times every 4 to 21 days. An appropriate carrier may also be used during immunization with sensitizing antigens.

After such an immunization, an increase in the level of desired antibodies in the serum is confirmed, and immunocytes are obtained from the mammals and subjected to cell fusion. Immunocytes that are preferably subjected to cell fusion are splenocytes in particular.

Regarding the other cells to be fused with the aforementioned immunocytes, mammalian myeloma cells used as parent cells include various known cell lines, such as P3X63Ag8.653 (Kearney, J. F. et al. J. Immunol. (1979) 123: 1548-1550), P3X63Ag8U.1 (Current Topics in Microbiology and Immunology (1978) 81: 1-7), NS-1 (Kohler. G and Milstein, C. Eur. J. Immunol. (1976) 6: 511-519), MPC-11 (Margulies. D. H. et al., Cell (1976) 8: 405-415), SP2/0 (Shulman, M. et al., Nature (1978) 276: 269-270), FO (de St. Groth, S. F. et al., J. Immunol. Methods (1980) 35: 1-21), S194 (Trowbridge, I. S. J. Exp. Med. (1978) 148: 313-323), and R210 (Galfre, G et al., Nature (1979) 277: 131-133).

In general, the above-mentioned immunocytes and myeloma cells may be fused according to standard methods, examples of which are described by Milstein *et al.* (Kohler. G and Milstein, C., Methods Enzymol. (1981) 73: 3-46).

More specifically, the above-mentioned cell fusion is carried out, for example, in a standard nutrient medium in the presence of a cell fusion promoting agent. For example, polyethylene glycol (PEG), Sendai virus (HVJ), or such can be used as the fusion promoting agent. If desired, adjuvants such as dimethylsulfoxide can additionally be used to increase fusion efficiency.

Regarding the proportion of immunocytes and myeloma cells used, an example of a preferred ratio of myeloma cells to immunocytes is from 1:1 to 1:10. The medium used for the aforementioned cell fusion may be, for example, RPMI 1640 medium, MEM medium, and such, which are suitable for growth of the aforementioned myeloma cell lines, or other kinds of medium commonly used for such cell culturing. Serum supplements, such as fetal calf serum (FCS), may also be used in combination.

The cell fusion is carried out by thoroughly mixing prescribed amounts of the aforementioned immunocytes and myeloma cells in the above-mentioned medium, adding to the medium a solution of PEG preheated to about 37°C generally at a concentration of 30% to 60% (w/v), wherein the PEG has an average molecular weight of approximately 1,000-6,000, for example, and mixing them to form the desired fusion cells (hybridomas). A suitable medium is then successively added. Cell fusing agents and such that are undesirable for the growth of hybridomas can be removed by repeatedly removing the supernatant by centrifugation.

The hybridomas are selected by culturing them in a common selection medium such as HAT medium (a medium containing hypoxanthine, aminopterin, and thymidine). Culturing in the HAT medium is continued for a sufficient time, usually from a few days to a few weeks, to allow death of all cells but the target hybridomas (the non-fused cells). The usual limiting dilution method is then performed to screen and clone hybridomas producing the target antibodies.

Alternatively, in addition to obtaining above-described hybridomas by using an antigen to immunize non-human animals, human lymphocytes can be sensitized *in vitro* with a desired antigen protein, or with cells expressing an antigen, and the sensitized B lymphocytes can be fused with human myeloma cells, such as U266, to obtain the desired human antibody with antigen-binding activity (see Japanese Patent Application Kokoku Publication No. (JP-B) H01-59878 (examined, approved Japanese patent application published for opposition)). Further, a desired human antibody can be obtained by administering a desired antigen or cells expressing an antigen to transgenic animals that have a repertoire of human antibody genes as above-described methods (see International Patent Application No. WO 93/12227, WO 92/03918, WO 94/02602, WO 94/25585, WO 96/34096, and WO 96/33735).

The hybridomas prepared in this manner that produce the monoclonal antibodies may be subcultured in a common medium, and then stored for a long time in liquid nitrogen.

Monoclonal antibodies may be obtained from the hybridomas using conventional techniques; for example, the hybridomas are cultured according to standard methods and the antibodies may be obtained from the culture supernatants. Alternatively, the hybridomas are administered to a compatible mammal for proliferation and then the antibodies may be obtained from the ascites fluid. The former method is suitable for obtaining highly pure antibodies, while the latter method is more suitable for mass production of antibodies.

Recombinant antibodies produced by genetic engineering techniques can be used as the monoclonal antibodies of the present invention. They can be produced by cloning an antibody gene from a hybridoma; incorporating the antibody gene into an appropriate vector, and introducing the vector into a host (see, for example, Borrebaeck, C. A. K., and Larrick, J. W, THERAPEUTIC MONOCLONAL ANTIBODIES, Published in the United Kingdom by MACMILLAN PUBLISHERS LTD, 1990).

Specifically, mRNAs encoding the variable (V) regions of antibodies are isolated from cells producing the target antibodies, such as hybridomas, by known methods, for example, by preparing total RNAs using guanidine ultracentrifugation methods (Chirgwin, J. M. et al., Biochemistry (1979) 18: 5294-5299), AGPC methods (Chomczynski, P. et al., Anal. Biochem. (1987) 162: 156-159), or such, and then preparing mRNAs using an mRNA Purification Kit (manufactured by Pharmacia) or such. The mRNAs can also be prepared directly by using a QuickPrep mRNA Purification Kit (manufactured by Pharmacia).

The obtained mRNAs are used to synthesize cDNAs of the antibody V regions using reverse transcriptase. cDNAs may be synthesized using the AMV Reverse Transcriptase First-strand cDNA Synthesis Kit. Alternatively, cDNA may be synthesized and amplified following the 5'-RACE method (Frohman, M. A. et al., Proc. Natl. Acad. Sci. USA (1988) 85: 8998-9002; Belyavsky, A. et al., Nucleic Acids Res. (1989) 17: 2919-2932) that uses the 5'-Ampli FINDER RACE Kit (manufactured by Clontech), and PCR. A desired DNA fragment is purified from the obtained PCR products and linked to a vector DNA. From this, a recombinant vector is produced. The recombinant vector is then used to transform *E*. *coli* and such, and the desired recombinant vector is prepared from a selected colony. The nucleotide sequence of the desired DNA may then be identified through known methods, such as the deoxy method.

When DNAs encoding a V region of a desired antibody are obtained, they are linked to DNAs encoding a constant region (C region) of a desired antibody, and then these are incorporated into expression vectors. Alternatively, DNAs encoding an antibody V region can be incorporated into expression vectors that include DNAs of an antibody C region.

Antibodies to be used in the present invention are produced by incorporating the antibody gene into an expression vector so that it will be expressed under the control of an expression regulatory region, such as an enhancer or promoter, as described hereafter. Antibodies can be subsequently expressed by transforming host cells with this expression vector.

In the present invention, artificially modified genetically-recombinant antibodies, for example, chimeric, humanized, and human antibodies may be used to reduce heterologous antigenicity against humans, or such. These modified antibodies can be produced using known methods.

The chimeric antibody can be produced by linking a DNA encoding the antibody V regions obtained as described above with a DNA encoding the C regions of the human antibody, incorporating this into an expression vector, and then introducing the vector into a host (see European Patent Application No. EP 125023 and International Patent Application No. WO 92/19759). Chimeric antibodies useful for the present invention can be obtained using this known method.

Humanized antibodies are also referred to as "reshaped human antibodies" or "humanized-type antibody". Such humanized antibodies are obtained by grafting the complementarity determining region (CDR) of an antibody derived from a non-human mammal, for example, a mouse, to the CDR of a human antibody. Such general gene recombination procedures are also known (see European Patent Application No. EP 125023 and International Patent Application No. WO 92/19759).

Specifically, a DNA sequence designed to link a murine antibody CDR to the framework region (FR) of a human antibody is synthesized by PCR, using several oligonucleotides produced to contain overlapping portions in the terminal regions. The obtained DNA is linked to a DNA encoding a human antibody C region, and incorporated into an expression vector. The antibody is produced by introducing this vector into a host (see European Patent Application No. EP 239400 and International Patent Application No. WO 92/19759).

The human antibody FR to be linked *via* CDR is selected such that the complementarity determining region forms a favorable antigen-binding site. In order for the CDR of the reshaped human antibody to form a suitable antigen-binding site, the amino acids in the framework region of the antibody variable region may be substituted as necessary (Sato, K. et al., Cancer Res. (1993) 53: 851-856).

Human antibody C regions are used in chimeric antibodies and humanized antibodies. An example of a human antibody C region is Cγ; thus, Cγ1, Cγ2, Cγ3, or Cγ4 may be used. In addition, to improve the stability of antibodies or the production thereof, the human antibody C region may be modified.

A chimeric antibody includes the variable region of an antibody derived from a non-human mammal and the C-region derived from a human antibody. A humanized antibody is composed of the CDR of an antibody derived from a non-human mammal and a framework region and C region derived from a human antibody. Since the antigenicity of these antibodies is low in a human body, they are suitable as antibodies of the present invention.

Known techniques for obtaining human antibodies include, in addition to those described above, panning using a human antibody library. For example, variable regions of human antibodies can be expressed as single-chain antibodies (scFvs) on the surface of phages using phage display methods, from which phages that bind to antigens can also be selected. DNA sequences encoding the variable regions of human antibodies that bind to the antigens can be determined by analyzing the genes of the selected phages. By revealing the DNA sequences of the scFvs that bind to the antigens, appropriate expression vectors carrying the sequences can be produced to yield human antibodies. These methods are already known, and the following publications can be referred to: WO 92/01047, WO 92/20791, WO 93/06213, WO 93/11236, WO 93/19172, WO 95/01438, and WO 95/15388.

The antibody gene constructed as described above can be expressed and obtained using known methods. When mammalian cells are used, such an antibody gene can be expressed using a DNA in which a common useful promoter, the antibody gene to be expressed, and a poly A signal positioned downstream of the gene on the 3' side are operably linked, or using a vector carrying the DNA. An example of a promoter/enhancer is the human cytomegalovirus immediate early promoter/enhancer.

Furthermore, as a promoter/enhancer that can be used for the expression of an antibody of the present invention, viral promoter/enhancers of retroviruses, polyomaviruses, adenoviruses, simian virus 40 (SV40), or such, or mammalian cell-derived promoter/enhancers such as human elongation factor 1α (HEF1α) or such can be used.

Antibody expression can be easily carried out by following, for example, the method of Mulligan *et al.* (Mulligan, R. C. et al., Nature (1979) 277: 108-114), using the SV40 promoter/enhancer, or the method of Mizushima *et al.* (Mizushima, S. and Nagata, S. Nucleic Acids Res. (1990) 18: 5322), using the HEF1α promoter/enhancer.

In the case of *E*. *coli,* the antibody can be expressed by an operably linked common useful promoter, a signal sequence for antibody secretion, and an antibody gene to be expressed. Examples of promoters include a lacZ promoter and an araB promoter. A lacZ promoter can be used according to the method of Ward *et al.* (Ward, E. S. et al., Nature (1989) 341: 544-546; Ward, E. S. et al., FASEB J. (1992) 6: 2422-2427) and an araB promoter can be used according to the method of Better *et al.* (Better, M. et al., Science (1988) 240: 1041-1043).

When antibody production is carried out in the periplasm of *E*. *coli,* the pelB signal sequence (Lei, S. P. et al. J. Bacteriol. (1987) 169: 4379-4383) may be used as a signal sequence for antibody secretion. After antibodies produced in the periplasm are separated, the antibody structure is appropriately refolded and then used (see for example WO 96/30394).

The replication origin may derive from SV40, polyoma viruses, adenoviruses, bovine papilloma viruses (BPV), and such. Furthermore, to increase the gene copy number in the host cells, the expression vector may contain, as a selection marker, an aminoglycoside phosphotransferase (APH) gene, thymidine kinase (TK) gene, *E. coli* xanthine guanine phosphoribosyl transferase (Ecogpt) gene, dihydrofolate reductase (dhfr) gene, and such.

Any production system may be used to produce the antibodies of the present invention. *In vitro* and *in vivo* production systems are available for antibody production systems. Production systems that use eukaryotic cells or prokaryotic cells are examples of *in vitro* production systems.

Production systems that use animal cells, plant cells, or fungal cells are available when using eukaryotic cells. Known animal cells include (1) mammalian cells, for example, CHO, COS, myeloma, baby hamster kidney (BHK), HeLa, Vero, and such, (2) amphibian cells such as *Xenopus laevis* oocytes, and (3) insect cells such as sf9, sf21, Tn5, and such. Known plant cells include *Nicotiana tabacum*-derived cells and these cells may be cultured as calluses. Known fungal cells include yeast, for example, the genus Saccharomyces, such as *Saccharomyces cerevisiae;* and filamentous fungi, for example, the genus Aspergillus such as *Aspergillus niger.*

Production systems that use bacterial cells are available when using prokaryotic cells. Examples of bacterial cells include *E. coli* and *Bacillus subtilis.*

Antibodies can be obtained by introducing the antibody gene of interest into these cells by transformation, then culturing these transformants *in vitro.* Transformants can be cultured using known methods. For example, DMEM, MEM, RPMI 1640, or IMDM may be used as a culture medium, and this may be used with serum supplements such as fetal calf serum (FCS). Furthermore, antibodies may be produced *in vivo* by transferring the antibody gene-introduced cells into the peritoneal cavity or such of the animals.

On the other hand, *in vivo* production systems include production systems using animals and production systems using plants. Mammals, insects and the like are used for production systems using animals.

Mammals such as goat, pig, sheep, mice, and cattle may be used (Vicki Glaser, SPECTRUM Biotechnology Applications, 1993). Alternatively, insects such as silkworms may be used. Tobacco, for example, can be used when using plants.

Antibody genes are introduced into such animals or plants, upon which the antibodies are produced in the body of the animals or plants and then recovered. For example, an antibody gene is prepared as a fusion gene by inserting the antibody gene into a gene encoding a protein that is specifically produced in milk, such as the goat β-casein gene. DNA fragments containing the fusion gene to which the antibody gene has been inserted are then injected into goat embryos, which are then introduced into female goats. The desired antibody can then be obtained from milk produced by the transgenic goats, which are born from the goats that received the embryos, or from their offspring. Hormones may be suitably administered to the transgenic goats to increase the production of milk containing the antibody of interest (Ebert, K.M. et al., Bio/Technology (1994) 12: 699-702).

When silkworm is used, baculoviruses carrying an antibody gene of interest is used to infect silkworms, whereupon the antibody of interest is obtained from their body fluids (Maeda, S. et al., Nature (1985) 315: 592-594). When tobacco is used, an antibody gene of interest is inserted into a plant expression vector, for example, pMON 530, and the vector may then be introduced into a bacterium, such as *Agrobacterium tumefaciens.* The bacteria are then used to infect tobacco, such as *Nicotiana tabacum*, whereupon the desired antibodies are obtained from the leaves of this tobacco (Julian K.-C. Ma et al., Eur. J. Immunol. (1994) 24: 131-138).

When producing an antibody using an *in vitro* or *in vivo* production system as described above, a DNA encoding the heavy chain (H chain) or the light chain (L chain) of the antibody can be each separately incorporated into a expression vector to simultaneously transform the host cell, or alternatively a DNA encoding the H chain and the L chain can be incorporated into a single expression vector to transform the host cell (see International Patent Application No. WO 94/11523).

The antibodies produced and expressed as described above can be isolated from the inside or outside the cell or from the host, and homogeneously-purified. The antibodies used in the present invention can be isolated and/or purified by affinity chromatography. Columns to be used for the affinity chromatography include, for example, protein A columns and protein G columns. Carriers used for the protein A columns include, for example, HyperD, POROS, and Sepharose FF. In addition, other conventional protein isolation and/or purification methods may be used. There is no limitation on the methods.

For example, the antibodies used in the present invention can be isolated and/or purified by appropriately selecting and combining chromatography other than the above-described affinity chromatography, filters, ultrafiltration, salting-out, dialysis, and such. Chromatography includes, for example, ion-exchange chromatography, hydrophobic chromatography, and gel filtration. Such chromatography can be applied to high performance liquid chromatography (HPLC). Alternatively, it is possible to use reverse phase HPLC.

The concentrations of the antibodies prepared as described above can be determined by absorbance measurement, ELISA, or such. Specifically, concentration is determined by appropriately diluting the antibody with PBS(-), measuring absorbance at 280 nm, and calculating the concentration by absorbance (1.35 OD = 1 mg/ml). Alternatively, when using ELISA, the measurement can be performed as described below. Specifically, 100 µl of 1 µg/ml goat anti-human IgG (manufactured by TAG) diluted with 0.1 M bicarbonate buffer (pH 9.6) is added to a 96-well plate (manufactured by Nunc) and incubated overnight at 4°C to immobilize the antibody. After blocking, 100 µl of an appropriately diluted antibody of the present invention or a sample including the antibody, or human IgG (manufactured by CAPPEL) as a standard, are added. The plate is incubated for one hour at room temperature.

After washing, 100 µl of 5,000-fold diluted alkaline phosphatase-labeled anti-human IgG (manufactured by BIO SOURCE) is added, and the plate is incubated for one hour at room temperature. After washing, a substrate solution is added to the plate and incubated. The concentration of the antibody of interest is calculated by measuring the absorbance at 405 nm using a Microplate Reader Model 3550 (manufactured by Bio-Rad).

Herein, the "aptamer" refers to nucleic acids that bind to various molecules such as proteins and hormones. An M-CSF receptor aptamer refers to a nucleic acid that binds to M-CSF receptor. An M-CSF receptor aptamer inhibitor refers to a nucleic acid that binds to M-CSF receptor and thereby inhibits the binding of M-CSF to M-CSF receptor. Such an M-CSF receptor aptamer may be an RNA or DNA. The RNA and DNA are not particularly limited as long as they bind to M-CSF receptor. Alternatively, nucleic acids may be modified in their riboses, phosphate backbones, or nucleobases, or at their both ends, and these nucleic acids are not particularly limited as long as they bind to M-CSF receptor. The form of nucleic acids may be double- or single-strand; however, single-stranded nucleic acids are preferred.

The length of aptamer is not particularly limited as long as it has a sufficient length for specifically binding to the target molecule. The length of aptamer is, for example, 10 to 200 nucleotides, preferably 10 to 100 nucleotides, more preferably 15 to 80 nucleotides, and still more preferably 15 to 50 nucleotides.

The aptamer constituted by nucleotides alone can be used as a therapeutic agent; however, it is possible to use aptamers linked to other molecules, for example, polyethylene glycol, cholesterol, peptides, liposomes, fluorescent dyes, radioisotopes, toxins, and other aptamers. Herein, the "aptamer" also includes such aptamers linked to other molecules.

Aptamers of the present invention can be selected using methods known to those skilled in the art. The aptamers can be selected, for example, by a Systematic Evolution ofLigands by Exponential Enrichment (SELEX) method (Tuerk, C. and Gold, L., 1990, Science, 249: 505-510), but the methods are not limited thereto. In the SELEX method, a target substance is mixed with a nucleic acid pool including approximately 10¹⁵ different nucleotide sequences to select nucleic acids that bind or more strongly bind to the target substance. The selected nucleic acids are amplified by RT-PCR or PCR, and then used as templates in the next round. Aptamers of interest can be obtained by repeating the above-described process about ten times.

When aptamers are used as pharmaceutical agents, they should be minimized and stabilized. Specifically, nucleotides that do not affect the aptamer activity are removed to minimize the aptamer, and some modifications are introduced to stabilize the aptamer. The half-life of natural RNA in serum is a few seconds; however, the half life is prolonged to one week or more by o-methylating the ribose at position 2' and linking inverted dT to both ends.

"Antisense RNA against M-CSF receptor" of the present invention includes antisense RNA complementary to a transcript of DNA encoding M-CSF receptor. There are a number of causes for the action of antisense nucleic acids in inhibiting target gene expression, including: inhibition of transcription initiation by triplex formation;

transcription inhibition by hybrid formation at a site with a local open loop structure generated by an RNA polymerase;
transcription inhibition by hybrid formation with the RNA being synthesized;
splicing inhibition by hybrid formation at an intron-exon junction;
splicing inhibition by hybrid formation at the site of spliceosome formation;
inhibition of transport from the nucleus to the cytoplasm by hybrid formation with mRNA;
splicing inhibition by hybrid formation at the capping site or poly(A) addition site;
inhibition of translation initiation by hybrid formation at the translation initiation factor binding site;
inhibition of translation by hybrid formation at the ribosome binding site adjacent to the initiation codon;
inhibition of peptide chain elongation by hybrid formation in the translational region of mRNA

or at the polysome binding site of mRNA; and
inhibition of gene expression by hybrid formation at the protein-nucleic acid interaction sites.
These inhibit the expression of target genes by inhibiting transcription, splicing, or translation.

The antisense sequences of the present invention may inhibit the expression of target genes, based on any of the actions described above. In one embodiment, antisense sequences designed to be complementary to an untranslated region adjacent to the 5' end of an mRNA for an M-CSF receptor gene may be effective for inhibiting translation of the gene. However, sequences complementary to a coding region or 3'-untranslated region can be used. Thus, the antisense DNAs to be used in the present invention include not only DNAs comprising sequences antisense to the coding regions, but also DNAs comprising sequences antisense to untranslated regions of the genes. Such antisense DNAs to be used are linked downstream of adequate promoters and are preferably linked with transcription termination signals on the 3' side. DNAs thus prepared can be introduced into desired plants using known methods. The sequences of the antisense DNAs are preferably complementary to a gene or portion thereof that is endogenous to the plants to be transformed with them. However, the sequences need not be perfectly complementary, as long as the antisense DNAs can effectively suppress expression of a gene. The transcribed RNAs preferably have 90% or higher, and most preferably 95% or higher complementarity to the target gene transcript. To effectively inhibit target gene expression using antisense sequences, the antisense DNAs are preferably at least 15 nucleotides or more, preferably 100 nucleotides or more, and more preferably 500 nucleotides or more. Generally, the length of the antisense DNAs to be used is less than 5 kb, preferably less than 2.5 kb.

Herein, the "dsRNA against M-CSF receptor" refers to double-stranded RNA that suppresses the expression of the M-CSF receptor gene through RNA interference (RNAi). RNA interference is a phenomenon where the expression of both of introduced foreign gene and endogenous target gene is suppressed when a double-stranded RNA (dsRNA) having a sequence that is identical with or similar to the target gene sequence is introduced into cells. When approximately 40 to several hundred base pairs of dsRNAs are introduced into cells, an RNase III-like nuclease called Dicer, which has a helicase domain, cleaves the dsRNAs from their 3' end into about 21-23 base pairs of short interference RNAs (siRNAs) in the presence of ATP. Specific proteins bind to the siRNAs to form a nuclease complex (RNA-induced silencing complex (RISC)). The complex recognizes the same sequence as siRNA and binds to a mRNA, and then cleaves the mRNA of the target gene at the position corresponding to the center of the siRNA by the RNaseIII-like enzymatic activity. Furthermore, aside from this pathway, the antisense strand of siRNA binds to mRNA and serves as a primer for RNA-dependent RNA polymerase (RdRP) to synthesize dsRNA. Another pathway is also considered where this dsRNA also serves as a substrate for Dicer, producing new siRNAs to amplify the effect.

The dsRNAs of the present invention include siRNAs and shRNAs. "siRNA" refers to a double-stranded RNA with short strands that are nontoxic within the cell. For example, the length of an siRNA can be 15 to 49 base pairs, preferably 15 to 35 base pairs, and still more preferably 21 to 30 base pairs. Alternatively, the length of the final double-stranded RNA portion that results from transcription of an siRNA to be expressed, can be 15 to 49 base pairs, preferably 15 to 35 base pairs, and more preferably 21 to 30 base pairs, for example. Meanwhile, shRNA refers to an siRNA where a single-stranded RNA forms a duplex *via* a hairpin structure.

The dsRNAs are not necessarily perfectly identical to the target gene; however, they have at least 70% or more, preferably 80% or more, more preferably 90% or more, and still more preferably 95% or more sequence identity.

The double-stranded RNA portions in dsRNAs, in which RNAs are paired, are not limited to those that are completely paired, but may include unpaired portions due to a mismatch (corresponding bases are not complementary), a bulge (there is no corresponding base on one strand) or the like. In the present invention, both bulges and mismatches may be included in double-stranded RNA regions where RNAs are paired with each other in dsRNAs.

Herein, the "ribozyme against M-CSF receptor" refers to a nucleic acid with a catalytic activity that affects the expression and function of the M-CSF receptor, and includes nucleic acids that specifically cleave the M-CSF receptor mRNA.

Moreover, M-CSF receptor inhibitors that suppress the expression or activity of M-CSF receptor can be obtained by using the screening methods described hereinbelow.

Herein, "leukemia" refers to suppression or prevention of leukemia symptoms and symptoms that occur in association with leukemia.

Herein, "leukemia" is a collective term for diseases that appear in blood where neoplastic hemopoietic cells multiply unlimitedly. Leukemia in which the cancer cells have lost differentiation ability is called acute leukemia, while leukemia in which the cancer cells retain differentiation ability is called chronic leukemia. In addition, when the origin of the cancer is a myeloid cell, the leukemia is grouped into myelocytic leukemia. Meanwhile, when the origin of the cancer is a lymphoid cell, the leukemia is grouped into lymphocytic leukemia. Thus, leukemia is roughly classified into four groups: acute myelogenous leukemia (AML), chronic myelogenous leukemia (CML), acute lymphoid leukemia (ALL), and chronic lymphoid leukemia (CLL). The classification also includes acute promyelocytic leukemia (APL), atypical leukemia (AUL), myelomonocytic leukemia (AMMoL), and juvenile chronic myelogenous leukemia (JCML). Furthermore, types of leukemia include acute monocytic leukemia (AMoL), chronic monocytic leukemia (CMoL), erythroleukemia, eosinophilic leukemia, basophilic leukemia, megakaryoblastic leukemia, plasma cell leukemia, chloroma, chronic neutrophilic leukemia, adult T-cell leukemia, lymphosarcoma cell leukemia, hairy cell leukemia, and prolymphocytic leukemia. There is no limitation on the type of leukemia in the present invention; however, preferred leukemia includes leukemia with expression of the MLL (Mixed Lineage Leukemia) fusion gene (MLL leukemia) and leukemia with expression of the MOZ (Monocytic Leukemia Zinc-finger) fusion gene (MOZ leukemia), which are intractable acute leukemia.

In MLL leukemia, the MLL gene is fused with other genes *vi*a specific chromosomal translocation, which is found in AML and ALL. The translocation partner includes 40 genes or more, including AF4, AF9, and p300/CBP. Furthermore, in AML, such gene fusion is found in M4/M5 (monocytic leukemia) of FAB classification. In addition, the increased expression of HOX group of genes is known in MLL leukemia.

In MOZ leukemia, the MOZ gene is fused with p300/CBP, TIF2, or ASX *via* specific chromosomal translocation, which is found in AML. Furthermore, such gene fusion is found in M4/M5 (monocytic leukemia) of FAB classification. In addition, the increased expression of HOXA9, HOXA10, MEIS1, and FLT3 are known in MOZ leukemia.

The present invention's agents comprising a therapeutic agent for leukemia may contain pharmaceutically acceptable materials such as preservatives and stabilizers. Pharmaceutically acceptable materials mean those that can be co-administered with an above-described therapeutic agent, which material may or may not have a therapeutic effect for the above-described leukemia. Alternatively, the materials may not have the therapeutic effect against leukemia but produce an additional or synergistic stabilizing effect when used in combination with an M-CSF receptor inhibitor.

Examples of pharmaceutically acceptable materials include sterilized water, physiological saline, stabilizers, excipients, buffers, preservatives, surfactants, chelating agents (for example, EDTA), and binders and the like.

In the present invention, examples of surfactants include non-ionic surfactants. Typical examples include, sorbitan fatty acid esters such as sorbitan monocaprilate, sorbitan monolaurate, or sorbitan monopalmitate; glycerol fatty acid esters such as glycerol monocaprilate, glycerol monomyristate, or glycerol monostearate with HLB 6 to 18.

Anionic surfactants can also be listed as surfactants. Typical examples of anionic surfactants may include alkyl sulfate salts having an alkyl group of 10 to 18 carbon atoms, such as sodium cetyl sulfate, sodium lauryl sulfate, or sodium oleyl sulfate; polyoxyethylene alkylether sulfate salts whose average mole of ethyleneoxide added is two to four and the number of carbon atoms in the alkyl group is 10 to 18, such as sodium polyoxyethylene lauryl sulfate; alkyl sulfosuccinate ester salts of 8 to 18 carbon atoms in the alkyl group, such as sodium lauryl sulfosuccinate ester; naturally-occurring surfactants such as lecithin or glycerol lipid phosphate; sphingophospholipids such as sphingomyelin; and sucrose fatty acid esters of 12 to 18 carbon atoms in the fatty acid.

One or a combination of two or more of these surfactants can be added to the pharmaceutical compositions of the present invention. A preferred surfactant to be used in the formulation of the present invention is a polyoxyethylene sorbitan fatty acid ester, such as Polysorbate 20, 40, 60, 80, or such, wherein Polysorbate 20 and 80 are particularly preferred. Polyoxyethylene polyoxypropylene glycol represented by Poloxamer (for example, Pluronic F-68®) is also preferred.

Examples of buffers in the present invention include those such as phosphoric acid, citric acid buffer, acetic acid, malic acid, tartaric acid, succinic acid, lactic acid, calcium phosphate, gluconic acid, caprylic acid, deoxycholic acid, salicylic acid, triethanolamine, fumaric acid, and such, other organic acids and such, carbonic acid buffer, Tris buffer, histidine buffer, imidazole buffer and the like.

Solution formulations can be prepared by dissolving into aqueous buffers that are known in the field of solution formulation. The buffer concentration is generally 1-500 mM, preferably 5-100 mM, and even more preferably 10-20 mM.

The agents of the present invention may further include other low-molecular-weight polypeptides, proteins such as serum albumin, gelatin, and immunoglobulin, amino acids, sugars and carbohydrates such as polysaccharides and monosaccharides, and sugar alcohols.

Examples of sugars and carbohydrates, such as polysaccharides and monosaccharides, in the present invention include dextran, glucose, fructose, lactose, xylose, mannose, maltose, sucrose, trehalose, and raffinose.

Examples of sugar alcohols in the present invention include mannitol, sorbitol, inositol, and such.

When used as an aqueous solution for injection, the solution can be mixed with, for example, physiological saline and isotonic solutions that include glucose or other adjunctive agents such as D-sorbitol, D-mannose, D-mannitol, and sodium chloride. The solution may also be combined with appropriate solubilizing agents, such as alcohol (for example, ethanol), polyalcohol (for example, propylene glycol or PEG), or non-ionic surfactant (polysorbate 80 or HCO-50).

If desired, diluents, solubilizing agents, pH-adjusting agents, soothing agents, sulfur-containing reducing agents, and antioxidants may be included.

If necessary, the pharmaceutical agents can be contained within microcapsules (microcapsules made of hydroxymethylcellulose, gelatin, poly[methyl methacrylate], or such), or made into colloidal drug delivery systems (such as liposomes, albumin microspheres, microemulsion, nanoparticles, and nanocapsules) (see for example, "Remington's Pharmaceutical Science 16h edition", Oslo Ed., 1980). Methods for preparing the pharmaceutical agents as controlled-release pharmaceutical agents are also well known, and such methods may be applied to the present invention (Langer et al., J. Biomed. Mater. Res. (1981) 15: 167-277; Langer, Chem. Tech. (1982) 12: 98-105; U.S. Patent No. 3,773,919; European Patent (EP) Patent Application No. 58,481; Sidman et al., Biopolymers (1983) 22: 547-556; EP 133,988).

Pharmaceutically acceptable carriers used are suitably selected from those mentioned above or combinations thereof according to the dosage form, but are not limited thereto.

The present invention relates to methods of screening for pharmaceutical compositions for treating or preventing leukemia by suppressing the expression or activity of M-CSF receptor.

Herein, the phrase "suppressing the expression of M-CSF receptor" includes suppression of gene transcription and suppression of protein translation. It includes not only complete blocking but also reduction of DNA expression.

In the first embodiment of the screening methods of the present invention, first, a number of test compounds are contacted with M-CSF receptor.

The nucleotide sequence of cDNA of human M-CSF receptor to be used in the methods of the present invention and the amino acid sequence of M-CSF receptor encoded by the DNA is shown in SEQ ID NO: 1 and 2, respectively. The M-CSF receptor to be used in the methods of the present invention also includes proteins functionally equivalent to the above-described known M-CSF receptor. Such proteins include, for example, mutants, alleles, variants and homologs of M-CSF receptor, partial peptides of M-CSF receptor, and fusion proteins with other proteins, but are not limited thereto.

M-CSF receptor mutants of the present invention include naturally-occurring proteins comprising an amino acid sequence with substitutions, deletions, insertions, and/or additions of one or more amino acids in the amino acid sequence of SEQ ID NO: 2, which are functionally equivalent to the protein comprising the amino acid sequence of SEQ ID NO: 2. The M-CSF receptor mutants also include proteins encoded by naturally-occurring DNAs that hybridize under stringent conditions to the DNA comprising the nucleotide sequence of SEQ ID NO: 1, which are functionally equivalent to the protein comprising the amino acid sequence of SEQ ID NO: 2.

In the present invention, the number of amino acids that are mutated is not particularly limited, but is generally 30 amino acids or less, preferably 15 amino acids or less, and more preferably 5 amino acids or less (for example, 3 amino acids or less). Preferably, the mutated amino acids conserve the properties of the amino acid side chain from the amino acids that were mutated. Examples of amino acid side chain properties include: hydrophobic amino acids (A, I, L, M, F, P, W, Y, and V), hydrophilic amino acids (R, D, N, C, E, Q, G, H, K, S, and T), amino acids having the following side chains: aliphatic side chains (G, A, V, L, I, and P); hydroxyl-containing side chains (S, T, and Y); sulfur-containing side chains (C and M); carboxylic acid- and amide-containing side chains (D, N, E, and Q); basic side chains (R, K, and H); and aromatic ring-containing side chains (H, F, Y, and W) (amino acids are represented by one-letter codes in parentheses). Polypeptides having a modified amino acid sequence, in which one or more amino acid residues are deleted, added, and/or substituted with other amino acids, are known to retain their original biological activities.

Herein, "functionally equivalent" means that a protein of interest has biological or biochemical functions equivalent to that of M-CSF receptor. Herein, the biological and biochemical functions of M-CSF receptor include a tyrosine phosphorylating ability (phosphorylation of STAT5). The biological properties also include the specificity of expression site and expression level.

Methods for preparing DNA encoding a "protein functionally equivalent" to a protein of interest are known to those skilled in the art, and include methods using techniques of hybridization and polymerase chain reaction (PCR). Specifically, those skilled in the art can readily isolate DNA having high homology to M-CSF receptor using as a probe the nucleotide sequence of M-CSF receptor (SEQ ID NO: 1) or a portion thereof, or using as primers oligonucleotides that specifically hybridize to M-CSF receptor (SEQ ID NO: 1). Thus, the DNAs of the present invention also include DNAs encoding proteins having a function equivalent to that of M-CSF receptor, which can be isolated using hybridization or PCR techniques.

In order to isolate such DNAs, hybridization reaction is preferably carried out under stringent conditions. Herein, the stringent conditions refers to "6 M urea, 0.4% SDS, 0.5x SSC" or a hybridization condition with a stringency equivalent thereto. Isolation of DNAs with higher homology can be expected using conditions with higher stringency, for example, 6 M urea, 0.4% SDS, and 0.1x SSC. The amino acid sequences encoded by the DNAs isolated as described above are expected to have a high homology to the amino acid sequence of a protein of interest. "High homology" refers to a sequence identity of at least 50% or more, more preferably 70% or more, and still more preferably 90% or more (for example, 95%, 96%, 97%, 98%, 99% or more) in the entire amino acid sequence. Such amino acid sequence identity and nucleotide sequence identity can be determined using the BLAST algorithm by Karin and Altschul (Proc. Natl. Acad. Sci. USA 87: 2264-2268, 1990; Proc. Natl. Acad. Sci. USA 90: 5873, 1993). Programs called BLASTN and BLASTX have been developed based on the BLAST algorithm (Altschul SF, et al., J. Mol. Biol. 215: 403, 1990). When nucleotide sequences are analyzed using BLASTN, parameters may be set at: score = 100 and wordlength = 12, for example. Alternatively, when amino acid sequences are analyzed using BLASTX, parameters may be set at: score = 50 and wordlength = 3, for example. When the BLAST and Gapped BLAST programs are used, it is possible to use default parameters for each program. Specific procedures for these analytical methods are known.

There is no limitation on the species from which M-CSF receptor to be used in the present invention is derived. Such species include, for example, human, monkey, mouse, rat, guinea pig, pig, bovine, yeast, and insect.

The form of M-CSF receptor to be used in the first embodiment is not particularly limited, and includes, for example, purified forms, intracellularly-expressed forms, and forms expressed in cell extracts.

M-CSF receptor can be purified by known methods. Cells expressing M-CSF receptor include cells expressing endogenous or exogenous M-CSF receptors. The above-described cells expressing endogenous M-CSF receptor include cultured cells, but are not limited thereto. The above-described cultured cells are not particularly limited, and include, for example, commercially available cultured cells. The species from which cells expressing endogenous M-CSF receptor are derived are not particularly limited, and include, for example, human, monkey, mouse, rat, guinea pig, pig, bovine, yeast, and insect. Meanwhile, the above-described cells expressing exogenous M-CSF receptor can be prepared, for example, by introducing a vector carrying a DNA encoding M-CSF receptor into cells. The vector can be introduced into cells by conventional methods, for example, calcium phosphate precipitation method, electroporation, lipofectamine method, microinjection, or the like. Alternatively, the above-described cells with exogenous M-CSF receptor can be prepared, for example, by integrating a DNA encoding M-CSF receptor into the chromosome by using a gene transfer method based on homologous recombination. Species from which cells to be introduced with exogenous M-CSF receptor are derived are not limited to mammals and include species for which techniques of expressing foreign protein into cells are established.

The cell extract expressing M-CSF receptor include, for example, a cell extract included in the *in vitro* transcription translation system to which a vector carrying a DNA encoding M-CSF receptor is added. The *in vitro* transcription translation system is not particularly limited, and commercially-available *in vitro* transcription translation kits or such can be used.

The "test compound" used in the methods of the present invention is not particularly limited, and include, for example, single compounds such as natural compounds, organic compounds, inorganic compounds, nucleic acids, proteins, and peptides; compound libraries, nucleic acid libraries, peptide libraries, expression products of gene libraries, cell extracts, cell culture supernatants, microbial fermentation products, marine organism extracts, plant extracts, prokaryotic cell extracts, eukaryotic cell extracts, and animal cell extracts. The test compounds are not particularly limited, and preferred examples include compounds that inhibit the M-CSF receptor-specific tyrosine kinase activity. If needed, the above-described test samples can be appropriately labeled and used. Such labels include, for example, radiolabels and fluorescent labels. In addition to the above-described test samples, they also include mixtures of two or more of these test samples.

In the present invention, the "contact" is carried out according to the state of M-CSF receptor. For example, when M-CSF receptor is purified, the "contact" can be achieved by adding test samples to the purified sample. Alternatively, when M-CSF receptor is endogenously-expressed or expressed in cell extracts, the "contact" can be achieved by adding test samples to cell culture supernatants or the cell extracts, respectively. Alternatively, when the test samples are proteins, the "contact" can be achieved, for example, by introducing a vector carrying a DNA encoding the proteins into cells expressing M-CSF receptor or by adding the vector to cell extracts expressing M-CSF receptor. In addition, for example, a two hybrid method using yeast, animal cells, or such can be used.

Then, in the first embodiment, the binding between M-CSF receptor and test compounds are detected. The detection method is not particularly limited. The binding between M-CSF receptor and a test compound can be detected, for example, by using a label (for example, a label that allows quantitative measurement, such as radiolabel or fluorescent label) attached to the test compound bound to the M-CSF receptor. Alternatively, labeling agents can be attached to the M-CSF receptor. The binding can also be detected by immobilizing the test samples or M-CSF receptor onto resins or chips. Alternatively, the binding can be detected using as an indicator an altered activity of M-CSF receptor resulting from the binding of test compounds to M-CSF receptor.

In this embodiment, the test compounds that bind to the M-CSF receptor are then selected. The selected compounds may include compounds that reduce the expression or activity of M-CSF receptor, which are expected to show a therapeutic or preventive effect against leukemia by suppressing the expression or activity of M-CSF receptor.

In the second embodiment of the screening method of the present invention, first, test compounds are contacted with cells expressing M-CSF receptor.

In the second embodiment, the expression level of M-CSF receptor is then determined. The expression level of M-CSF receptor can be determined by methods known to those skilled in the art. For example, the gene mRNA is extracted according to a conventional method, and then Northern hybridization or RT-PCR is carried out using the mRNA as a template to determine the transcriptional level of the gene. Alternatively, the expression level of the gene is determined using DNA array techniques.

Furthermore, fractions containing M-CSF receptor encoded by the gene are collected by a conventional method, and the expression of M-CSF receptor can be determined at the translational level by detecting M-CSF receptor expression using electrophoresis such as SDS-PAGE. Alternatively, the expression of M-CSF receptor at the translational level can be determined by Western blotting using an antibody against M-CSF receptor. The above-described antibodies can be used as an antibody against M-CSF receptor.

Then in the second embodiment, test compounds that reduce the expression level of M-CSF receptor as compared to when the test compounds are not contacted are selected. The selected compounds may include compounds that reduce the expression of M-CSF receptor, which are expected to show a therapeutic or preventive effect against leukemia by suppressing the expression of M-CSF receptor.

In the third embodiment of the screening method of the present invention, first, cells or a cell extract containing a DNA in which a reporter gene is operably linked to downstream of the promoter region of a DNA encoding M-CSF receptor is provided.

In the third embodiment, "operably linked" means that a reporter gene is linked to the promoter region of M-CSF receptor gene such that the expression of the reporter gene is induced by the binding of transcriptional factors to the promoter region of M-CSF receptor gene. Thus, even when the reporter gene is linked to another gene and produces a fusion protein with its gene product, such cases are also included in the definition of "operably linked", as long as the expression of the fusion protein is induced by the binding of transcriptional factors to the promoter region of the M-CSF receptor gene.

The above-described reporter gene is not particularly limited as long as the expression is detectable. The reporter gene includes, for example, CAT gene, lacZ gene, luciferase gene, β-glucuronidase gene (GUS), and the GFP gene, which are commonly used by those skilled in the art. The reporter gene also includes DNAs encoding M-CSF receptor.

Cells or cell extract containing DNA in which a reporter gene is operably linked to downstream of the promoter region of a DNA encoding M-CSF receptor can be prepared by the methods described in the first embodiment.

In the third embodiment, next, test samples are contacted with the above-described cells or cell extract. Then, the expression level of the above-described reporter gene in the cells or cell extract is determined.

The expression level of a reporter gene can be determined by methods known to those skilled in the art, depending on the type of reporter gene used. For example, when the reporter gene is the CAT gene, the expression level of the reporter gene can be determined by detecting the acetylation of chloramphenicol by the gene product. When the reporter gene is the lacZ gene, the expression level of the reporter gene can be determined by detecting the color development of a chromogenic compound due to the catalytic activity of the gene expression product. When the reporter gene is the luciferase gene, the expression level of the reporter gene can be determined by detecting the fluorescence of a fluorescent compound due to the catalytic activity of the gene expression product. When the reporter gene is the β-glucuronidase gene (GUS), the expression level of the reporter gene can be determined by detecting the luminescence of Glucuron (manufactured by ICN) or color development of 5-bromo-4-chloro-3-indolyl-β-glucuronide (X-Gluc) due to the catalytic activity of the gene expression product. When the reporter gene is the GFP gene, the expression level of the reporter gene can be determined by detecting the fluorescence of GFP protein.

Alternatively, when the M-CSF receptor gene is used as a reporter gene, the expression level of the gene can be determined by the methods described in the second embodiment.

In the third embodiment, then, test compounds that reduce or increase the expression level of the reporter gene as compared to when the test compounds are not contacted are selected. The selected compounds may include compounds that reduce the expression of M-CSF receptor, which are expected to show a therapeutic or preventive effect against leukemia by suppressing the expression of M-CSF receptor.

In the fourth embodiment of screening method of the present invention, first, test compounds are contacted with cells expressing the M-CSF receptor gene.

In the fourth embodiment, then, the activity of the above-described M-CSF receptor is determined. The activity of M-CSF receptor includes the tyrosine phosphorylating ability. Specifically, the activity of M-CSF receptor can be determined by measuring the phosphorylation of STAT5. Next, test compounds that reduce or increase the above-described activity as compared to when test compound are not contacted are selected. The selected compounds may include compounds that reduce the activity of M-CSF receptor, which are expected to show a therapeutic or preventive effect against leukemia by suppressing the activity of M-CSF receptor.

The present invention relates to methods for treating or preventing leukemia, which comprise the step of administering an M-CSF receptor inhibitor to subjects. The M-CSF receptor inhibitor may be administered in any one of remission-induction therapy, consolidation therapy, and maintenance and intensification therapy as a treatment of acute leukemia.

Herein, the definition "suppression of the expression of M-CSF receptor" includes suppression of gene transcription and suppression of protein translation. It refers to not only complete blocking of DNA expression but also decrease of expression.

When the M-CSF receptor inhibitors of the present invention are used as pharmaceutical agents for treating humans or other animals, the compounds themselves may be administered directly to patients or may be administered after formulating them by known pharmaceutical methods. When formulating the compounds, the above-described pharmaceutically acceptable materials can be added.

All agents of the present invention can be administered in the form of a pharmaceutical, and can be given orally or parenterally and systemically or topically. For example, intravenous injection such as drip infusion, intramuscular injection, intraperitoneal injection, subcutaneous injection, suppository, colonic infusion, or oral enteric coating agent may be selected, and a suitable administration method can be selected according to the age and symptoms of the patient. The effective dose can be selected from the range of 0.001 mg to 100 mg/kg body weight in each administration. Alternatively, the dosage can be selected from 0.1-1,000 mg per patient, or preferably 0.1-50 mg per patient. For example, for anti-M-CSF receptor or anti-M-CSF antibodies, preferred dose and method of administration are as follows: an amount that results in free antibody presence in blood is an effective dose for these antibodies, and specific examples include administration methods such as 0.5 mg to 40 mg per month (four weeks) per kg body weight, which is preferably one mg to 20 mg in one to several doses, for example, by methods including intravenous injection such as drip infusion or subcutaneous injection following an administration schedule of twice/week, once/week, once/two weeks, once/four weeks, or such. The administration schedule can be adjusted by extending the administration interval from twice/week or once/week to once/two weeks, once/three weeks, or once/four weeks by observing post-administration condition and changes in blood test values. The effective dose of M-CSF receptor aptamer is an amount that results in free aptamer presence in blood, and specific examples include administration methods such as 0.1 mg to 100 mg per month (four weeks) per kg body weight, which is preferably 0.1 mg to 40 mg in one to several doses, for example, by methods including intravenous injection such as drip infusion or subcutaneous injection following an administration schedule of twice/week, once/week, once/two weeks, once/four weeks, or such. When administered to humans, preferred doses and administration methods are determined based on an assessment using the animal model described below.

The present invention relates to methods for testing leukemia. Such methods include methods for testing leukemia, which comprise the step of determining the expression level of M-CSF receptor in cells expressing M-CSF receptor. The determination of the expression level of M-CSF receptor can be determined by the methods described above.

When the expression level of M-CSF receptor is elevated, the tyrosine phosphorylation is assumed to be enhanced in the neoplastic cells. Thus, the onset of leukemia can be tested by determining the expression level of M-CSF receptor.

The present invention relates to test agents for leukemia. "Substances that bind to M-CSF receptor" of the test agents are not particularly limited, as long as they bind to M-CSF receptor protein, M-CSF receptor gene region, or M-CSF receptor mRNA and include, for example, oligonucleotides that hybridize with the M-CSF receptor gene region or antibodies that recognize M-CSF receptor. Such oligonucleotides that hybridize with the M-CSF receptor gene region include, for example, oligonucleotides with a length of at least 15 nucleotides.

Oligonucleotides to be used as the test agents of the present invention include polynucleotides. The oligonucleotides of the present invention can be used as probes or primers for detecting or amplifying DNAs encoding the M-CSF receptor of the present invention, as probes or primers for detecting the expression of M-CSF receptor gene, or as nucleotides or nucleotide derivatives (for example, antisense oligonucleotides, ribozymes, and DNAs encoding them) for regulating the expression of the M-CSF receptor protein of the present invention. Alternatively, the oligonucleotides of the present invention may be used in a form of DNA array substrates.

When the oligonucleotides are used as primers, their length is typically 15 bp to 100 bp, preferably 15 bp to 30 bp. The primers are not particularly limited, as long as they are capable of amplifying at least a portion of a DNA of the present invention or the complementary strand thereof. When the oligonucleotides are used as primers, they may be designed to be complementary to their 3' end and attach restriction sites or tags at their 5' end.

Alternatively, when the above-described oligonucleotides are used as probes, they are not particularly limited as long as they specifically hybridize with at least a portion of an M-CSF receptor DNA of the present invention or the complementary strand thereof. The probes may be synthetic oligonucleotides, typically, with a length of at least 15 bp or more.

When the oligonucleotides of the present invention are used as probes, they are preferably used after appropriate labeling. Such labeling methods include, for example, methods of labeling the oligonucleotides with ³²P by phosphorylating their 5' end using T4 polynucleotide kinase, and methods of incorporating substrate nucleotides labeled with an isotope such as ³²P, fluorescent dye, biotin, or such, using random hexamer oligonucleotides or such as primers, and DNA polymerases such as the Klenow enzyme (random priming method, etc.).

The oligonucleotides of the present invention can be synthesized, for example, using a commercially available oligonucleotide synthesizer. The probes can also be prepared as double-stranded DNA fragments, which are obtained by restriction enzyme treatment or such.

Antibodies used as the test agents of the present invention are not particularly limited as long as they recognize the above-described M-CSF receptor, but antibodies that specifically recognize M-CSF receptor is preferred. The anti-M-CSF receptor antibodies include, for example, those described above.

Antibodies used in the present invention may be conjugated with various molecules such as polyethylene glycol (PEG), radioisotopes, and toxins. Such conjugated antibodies can be obtained by chemically-modifying the prepared antibodies. Methods of modifying antibodies have already been established in this field. The "antibodies" of the present invention include such conjugated antibodies.

The above-described test agents may comprise the above-described pharmaceutically acceptable materials, in addition to the oligonucleotides or antibodies, which are active ingredients.

All prior art documents cited herein are incorporated herein by reference.

### [Examples]

Herein below, the present invention will be specifically described with reference to Examples, but it is not to be constructed as being limited thereto.

### [Example 1]

To generate a mouse model for acute myelogenous leukemia (herein below, abbreviated as AML), c-KIT⁺ bone marrow (BM) cells were infected with MSCV-MOZ-TIF2-ires-EGFP retrovirus and transplanted into lethally-irradiated mice. AML mice models are generated as described (Yokoyama, A. et al., Cell (2005) 123: 207-18). The mice developed AML about two months after transplantation as reported by Deguchi *et al.* (Deguchi, K. et al., Cancer Cell (2003) 3: 259-271). When limited numbers ofBM cells derived from the AML mice were transplanted into irradiated mice, these mice developed AML three to six weeks after transplantation, suggesting that transplantable leukemia-initiating cells (herein after, abbreviated as LICs) were present in the BMs of AML mice (Huntly, B. J. et al., Cancer Cell (2004) 6: 587-96).

In order to identify LICs, the present inventors investigated the BM cells of the AML mice for various cell surface markers by fluorescence-activated cell sorting (FACS) analysis, and found that M-CSFR (described as CSF1R in the Drawings)^{high} and M-CSFR^{low/-} cells were present in the BMs of the AML mice (Figures 1A).

Both M-CSFR^{high} and M-CSFR^{low/-} cells expressed equivalent levels of MOZ-TIF2 proteins (Fig. 1B). To determine their LIC activity, M-CSFR^{high} and M-CSFR^{low/-} cells were isolated by a cell sorter, and limited numbers of cells (10-10⁴ cells) were transplanted into irradiated mice. One hundred of M-CSFR^{high} cells was sufficient to induce AML in all mice transplanted (Fig. 1C). Conversely, no mice developed AML after 10³ of M-CSFR^{low/-} cells were transplanted, and only half of the mice developed AML with delayed onset when 10⁴ of M-CSFR^{low/-} cells were transplanted (Fig. 1D). Thus, the M-CSFR^{high} cells exhibited more than 100 times stronger LIC activity compared with M-CSFR^{low/-} cells.

### [Example 2]

To determine if leukemogenesis can be suppressed by removing M-CSFR-expressing cells that exhibit stronger LIC activity, the present inventors used transgenic mice expressing a drug-inducible FKBP-Fas suicide gene and EGFP under the control of the M-CSFR promoter (Burnett, S. H. et al., J Leukoc Biol (2004) 75: 612-23) (Fig. 1E). In the transgenic mice, expression levels of endogenous M-CSFR were proportional to those of EGFP and FKBP-Fas (Fig. IF), and conditional ablation of M-CSFR-expressing cells can be induced by injection of the AP20187 dimerizer (Burnett, S. H. et al., J Leukoc Biol (2004) 75: 612-23). c-KIT⁺ BM cells of the transgenic mice were infected with the MOZ-TIF2 retrovirus, and transplanted into lethally-irradiated wild-type mice. These mice developed AML about two months after transplantation, and their BM cells (10⁵ cells/mouse) were transplanted into secondary recipient mice. Seven days after transplantation, the mice were injected with AP20187 as described in Burnett *et al.* (Burnett, S. H. et al., J Leukoc Biol (2004) 75: 612-23). AP20187 was a gift from Ariad Pharmaceuticals. AP20187 (10mg/kg) was administered intravenously daily for 5 days, and then 1 mg/kg AP20187 was administered every three days thereafter as described previously (Burnett, S. H. et al., J Leukoc Biol (2004) 75: 612-23). An increase in number of M-CSFR^{high} cells (Fig. 1G) and splenomegaly (Fig. 1H) were observed in the untreated mice three weeks after transplantation. However, neither M-CSFR^{high} cells nor splenomegaly were detected in the AP20187-treated mice. All untreated mice, and none of the AP20187-treated mice, developed AML 5-6 weeks after transplantation (Fig. 1I). These results indicate that leukemogenesis can be suppressed by removing M-CSFR-expressing cells and treatment targeting M-CSFR is effective.

### [Example 3]

Then, the present inventors investigated the M-CSFR expression in AML mice with MLL-AF10 (DiMartino, J. F. et al., Blood (2002) 99: 3780-59), and found that M-CSFR^{high} and M-CSFR^{low/-} cells were also present in BM of MLL-AF 10-induced AML mice (Fig. 2A). When transplanted, cell sorter-sorted M-CSFR^{high} cells exhibited more than 10 times stronger leukemia-initiating activity than M-CSFR^{low/-} cells (Figs. 2B and 2C). STAT5 and ERK, which are downstream effectors for M-CSFR, are activated in a variety of leukemia and myeloproliferative disorders. The present inventors investigated the phosphorylation status of these proteins in M-CSFR^{high} and M-CSFR^{low/-} cells from MOZ-TIF2- and MLL-AF10-induced AML mice using immunoblot analysis with phospho-specific anti-STAT5 and anti-ERK antibodies. STAT5 was highly phosphorylated in M-CSFR^{high} cells but not in M-CSFR^{low/-} cells (Fig. 2D), while ERK1/2 were phosphorylated in both M-CSFR^{high} and M-CSFR^{low/-} cells. These results suggest that STAT5 is specifically activated in the LICs of MOZ-TIF2- and MLL-AF10-induced AML mice.

### [Example 4]

Since MOZ-TIF2- and MLL-AF10-induced leukemia cells can form colonies in methylcellulose medium (Deguchi, K. et al., Cancer Cell (2003) 3: 259-71; DiMartino, J. F. et al., Blood (2002) 99: 3780-5), flow-sorted M-CSFR^{high} and M-CSFR^{low/-} cells were tested for their colony-forming ability in the presence of either M-CSF/CSF1 or multiple cytokines. Colony formation in methylcellulose medium, FACS analysis and cell sorting were performed as described (Katsumoto, T. et al., Genes Dev (2006) 20: 1321-30). As the present inventors expected, M-CSFR^{high} cells but not M-CSFR^{low/-} cells formed colonies in the CSF1 condition, suggesting that functional M-CSFR is expressed in M-CSFR^{high} cells (Fig. 2E). However, M-CSFR^{high} and M-CSFR^{low/-} cells formed equal number of colonies in the multiple cytokine condition (Fig. 2F). It has been known that upregulation of A-cluster Hox genes, including HoxA9, is important for both MLL translocation-mediated and MOZ translocation-mediated leukemogenesis (Dou, Y. & Hess, J. L. Int J Hematol (2008) 87: 10-8; Camos, M. et al., Cancer Res (2006) 66: 6947-54). Quantitative RT-PCR analysis showed that HoxA9 expression was upregulated in both M-CSFR^{high} and M-CSFR^{low/-} cells (Fig. 2H).

### [Example 5]

To determine if M-CSFR expression is also upregulated in human AML cells with MOZ- or MLL-translocations, the present inventors investigated M-CSFR expression in BM cells from AML patients with a t(8;16) translocation expressing *MOZ-CBP* (K.T. & E.T., submitted) and in THP-1 cells with a t(9;11) translocation expressing MLL-AF9 fusion proteins (Tsuchiya, S. et al., Int J Cancer (1980) 26: 171-6; Odero, M. D. et al., Genes Chromosomes Cancer (2000) 29: 333-8). FACS analysis indicated that M-CSFR^{high} and M-CSFR^{low/-} cells were also observed in both the AML cells with t(8;16) (Fig. 2I) and the THP-1 cells (Fig. 2J).

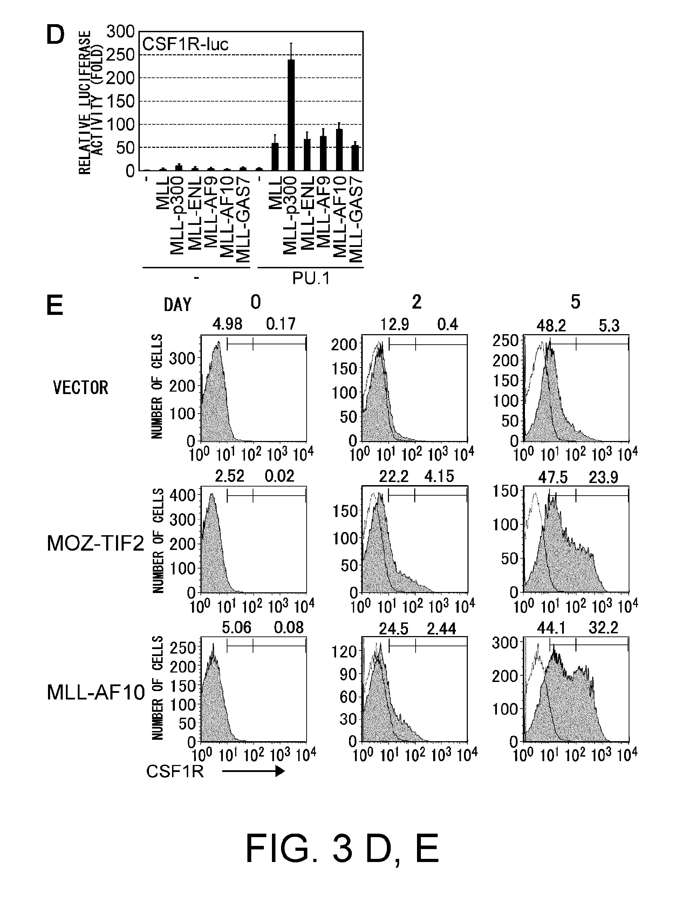

It is reported that monocyte-specific expression of M-CSFR is regulated by transcription factors such as AML1, PU.1, and C/EBP (Zhang, D. E. et al., Mol Cell Biol (1996) 16: 1231-40). The present inventors have found that MOZ interacts with AML1 and PU.1, but not with C/EBPα or C/EBPε, to stimulate transcriptions of their target genes (Kitabayashi, I. et al., Embo J (2001) 20: 7184-96; Katsumoto, T. et al., Genes Dev (2006) 20: 1321-30). Reporter analysis using an *M-CSFR* promoter-luciferase construct showed that MOZ, MOZ-CBP, and MOZ-TIF2 activated the *M-CSFR* promoter in the presence of PU.1 but not in the presence of AML1 (Fig. 3B). To investigate MLL-mediated transcription regulation, the present inventors tested the interaction of MLL with several hematopoietic transcription factors, including AML1, PU.1, c-Myb, C/EBPα, and C/EBPε, and found that MLL specifically interacts with PU.1 (Fig. 3C). MLL and MLL-fusions also stimulated PU.1-dependent activation of the *M-CSFR* promoter (Fig. 3D). MLL, MLL-p300, MOZ, and MOZ-CBP did not activate the *M-CSFR* promoter mutant lacking PU.1-binding sites (Zhang, D. E. et al., Mol Cell Biol (1994) 14: 373-81) (Fig. 4). These results suggest that MOZ, MLL, and their fusion proteins activate *M-CSFR* transcription in PU.1-dependent manners.

Immunoprecipitation, immunoblotting and reporter analysis were performed as described (Aikawa, Y. et al., Embo J (2006) 25: 3955-65). *M-CSFR*-luciferase reporter genes were generated by ligation of *M-CSFR* promoter (Zhang, D. E. et al., Mol Cell Biol (1994) 14: 373-81) with pGL4.

Deletion analysis showed that PU.1 interacts with the N-terminal and C-terminal regions of MOZ (Figs. 3A and 5) and the N-terminal region of MLL (Figs. 3A and 6B). Reporter analysis using MLL deletion mutants showed that the most C-terminal SET domain, which is needed for histone methyltransferase activity, are not required for the activation of M-CSFR by MLL (Fig. 6C). The acidic amino acid-rich region (DE region) or ETS domain of PU.1 were required for the high-affinity interaction of PU.1 with MOZ or MLL, respectively (Figs. 7B and 7C). The DE region and ETS domain were both required for the activation of PU.1-mediated transcription by MOZ, MLL, MOZ-CBP, and MLL-p300 (Fig. 7D).

### [Example 6]

To test if MOZ-TIF2 or MLL-AF10 stimulate PU.1-dependent induction of endogenous M-CSFR, the present inventors used PU.1^{-/-} myeloid progenitors expressing a PU.1-estrogen receptor fusion protein (PUER). The PUER cells can differentiate into macrophages upon restoration of PU.1 activity by exposure to 4-Hydroxytamoxifen (4-HT) (Walsh, J. C. et al., Immunity (2002) 17: 665-76). The PUER cells were infected with MSCV-MOZ-TIF2-ires-GFP, MSCV-MLL-AF10-ires-GFP, or control retroviruses. GFP⁺ cells were sorted and cultured in the presence of 4-HT. FACS analysis showed that two peaks indicating M-CSFR^{high} and M-CSFR^{low/-} cells could be detected in cells expressing MOZ-TIF2 or MLL-AF10, but only M-CSFR^{low/-} cells but not M-CSFR^{high} cells could be detected in vector control cells, five days after exposure to 4-HT (Fig. 3E). The two peaks in M-CSFR expression levels were similar to those observed in MOZ-TIF2- and MLL-AF10-induced AML mice as described above. Quantitative RT-PCR analysis showed that both MOZ-TIF2 and MLL-AF10 strongly stimulated PU.1-induced M-CSFR mRNA expression (Fig. 8).

### [Example 7]

To determine if PU.1 is essential for MOZ-TIF2- and MLL-AF 10-induced AML, PU.1^{+/+} and PU.1^{-/-} fetal liver cells of E12.5 littermates were infected with retroviruses for MOZ-TIF2, MLL-AF10, or N-Myc, and were transplanted into irradiated mice. Although the mice with PU.1^{+/+} cells expressing MOZ-TIF2 or MLL-AF10 developed AML 9-15 weeks after transplantation, mice with PU.1^{-/-} cells were quite healthy for at least six months (Figs. 3F and 3G). Thus, PU.1 is required for development of MOZ-TIF2- and MLL-AF10-induced AML. In contrast, all mice transplanted with either PU.1^{-/-} or PU.1^{+/+} cells expressing N-Myc developed AML 6-10 weeks after transplantation (Fig. 3H).

Taken together, the results indicated that MOZ and MLL, as well as their leukemia-associated fusion proteins, activate transcription of *M-CSFR* by binding to PU.1. MOZ- and MLL-fusions might constitutively stimulate high levels of M-CSFR expression to induce AML (Fig. 3I). Meanwhile, it is likely that normal MOZ and MLL control M-CSFR expression to an appropriate level to regulate normal hematopoiesis (Figure 3J). In fact, expression of M-CSFR was impaired in either MOZ-/- or MLL-/- fetal liver cells (Figs. 9A and 9B).

The results of the present invention suggest that M-CSFR could be a therapeutic target for *MLL and MOZ* leukemogenesis. It has been reported that M-CSFR is sensitive to the tyrosine kinase inhibitor Imatinib mesylate (STI571; Glivec^{®}) (Taylor, J. R. et al., Oncogene (2006) 25: 147-51; Dewar, A. L. et al., Cell Cycle (2005) 4: 851-3; Dewar, A. L. et al., Blood (2005) 105: 3127-32). The present inventors tested the effect of Imatinib on MOZ-TIF2- and MLL-AF10-induced AML in mice. Imatinib (100 mg/kg) was administrated orally twice a day. Oral administration of Imatinib slowed onsets of AML induced by either fusion protein (Figs. 10A and 10B), but did not affect the progress ofN-MYC-induced AML (Fig. 10C). MOZ-TIF2-induced splenomegaly was inhibited by treatment with Imatinib (Fig. 10D).

### [Example 8]

Herein, the present inventors showed that LICs were highly enriched in M-CSFR^{high} cells. FACS analysis indicated that the M-CSFR^{high}-LICs were c-Kit⁺ Sca-1⁻ CD16/32⁺ Mac-l^{low} Gr-1⁺ (Fig. 11). Cells of side population (SP), a feature of some normal and malignant stem cells, were present in the BMs of MOZ-TIF2-induced AML mice (Figs. 12A). While most SP cells were M-CSFR^{high} cells, non-SP cells contained both M-CSFR^{high} and M-CSFR^{low/-} cells (Fig. 12B). LICs were approximately 10 times enriched in the SP fraction compared with the non-SP fraction (Figs. 12C and 12D). Since population of SP cells were very small (approximately 0.12%), the absolute number of LICs in the SP fraction was small (1-2%), and most of the LICs were present in the non-SP fraction.

It has been suggested that second mutations, such as activating point mutations, in receptor tyrosine kinases (e.g., FLT3 and c-KIT) are required for the fusion genes such as AML1-ETO, PML-RARα, or CBFβ-MYH11 to induce acute leukemia (Gilliland, D. G., Semin Hematol (2002) 39: 6-11). In contrast, MLL- or MOZ-fusions alone can induce the rapid onset of AML (Deguchi, K. et al., Cancer Cell (2003) 3: 259-71; Corral, J. et al., Cell (1996) 85: 853-61; Forster, A. et al., Cancer Cell (2003) 3: 449-58; Lavau, C. et al., Embo J (1997) 16: 4226-37). Upregulation of *Hox* genes and the HOX cofactor *Meisl* is critical for LIC induction and maintenance, but it does not recapitulate all the biological and clinical features ofMLL leukemia and MOZ leukemia (Dou, Y. & Hess, J. L., Int J Hematol (2008) 87, 10-8; Jin, G. et al., Blood (2007) 109, 3998-4005). The present inventors' data demonstrated that MLL- and MOZ-fusions interact with PU.1 to induce the upregulation of the receptor tyrosine kinase M-CSFR expression, which results in activation of two classes of pathways to induce the early-onset of AML (Fig. 10E). They also provide M-CSFR as novel molecular targets for the treatment of these forms of leukemia (Figs. 1F-I, 11A-D).

### Industrial Applicability

The present invention revealed that the M-CSF receptor (M-CSFR, CSF1R, c-FMS, or CD115) was highly expressed in cells having the activity of inducing MLL or MOZ leukemia (leukemia stem cells), both of which are intractable acute leukemia.

Specifically, it is expected that MLL leukemia and MOZ leukemia can be treated by suppressing the expression or signal activity of M-CSF receptor, or by killing cells expressing M-CSF receptor. MLL leukemia, MOZ leukemia, and the like can be treated by using the therapeutic agents of the present invention for leukemia. Furthermore, leukemia can be diagnosed by determining the expression level of M-CSF receptor.

## Claims

1. A therapeutic agent for MLL leukemia or MOZ leukemia comprising an M-CSF receptor inhibitor as an active ingredient.

2. The therapeutic agent for leukemia of claim 1, wherein the M-CSF receptor inhibitor is an agent that inhibits tyrosine kinase activity of the M-CSF receptor.

3. The therapeutic agent of claim 1 for leukemia, wherein the M-CSF receptor inhibitor is an anti-M-CSF receptor antibody.

4. The therapeutic agent for leukemia of claim 1, wherein the M-CSF receptor inhibitor is an aptamer against the M-CSF receptor.

5. The therapeutic agent for leukemia of claim 1, wherein the M-CSF receptor inhibitor is an antisense RNA or dsRNA.

6. The therapeutic agent for leukemia of claim 1, wherein the M-CSF receptor inhibitor is Imatinib mesylate.

7. The therapeutic agent for leukemia of any one of claims 1 to 6, wherein the M-CSF receptor is any one of M-CSFR, CSF1R, c-FMS, or CD115.

8. A method of treating or preventing leukemia in a subject, which comprises the step of administering an M-CSF receptor inhibitor to the subject.

9. Use of an M-CSF receptor inhibitor in the manufacture of a therapeutic agent for leukemia.

10. A method of screening for a pharmaceutical composition that treats or prevents leukemia by suppressing an expression of an M-CSF receptor, which comprises the steps of:
(a) contacting a test compound with an M-CSF receptor;
(b) detecting the binding of a test compound to the M-CSF receptor; and
(c) selecting a test compound that binds to the M-CSF receptor.

11. A method of screening for a pharmaceutical composition that treats or prevents leukemia by suppressing the expression of an M-CSF receptor, which comprises the steps of:
(a) contacting a test compound with a cell expressing an M-CSF receptor gene;
(b) determining the expression level of the M-CSF receptor; and
(c) selecting a test compound that reduces the expression level of the M-CSF receptor as compared to when the test compound is not contacted.

12. A method of screening for a pharmaceutical composition that treats or prevents leukemia by suppressing the expression of an M-CSF receptor, which comprises the steps of:
(a) providing a cell or cell extract comprising a DNA wherein a reporter gene is operably linked to downstream of the promoter region of DNA encoding an M-CSF receptor;
(b) contacting a test compound with the cell or cell extract;
(c) determining the expression level of the reporter gene in the cell or cell extract; and
(d) selecting a test compound that reduces the expression level of the reporter gene as compared to when the test compound is not contacted.

13. A method of screening for a pharmaceutical composition that treats or prevents leukemia by suppressing the activity of an M-CSF receptor, which comprises the steps of:
(a) contacting a test compound with a cell expressing an M-CSF receptor;
(b) determining the activity of the M-CSF receptor in the cell; and
(c) selecting a test compound that reduces the activity of the reporter gene as compared to when the test compound is not contacted.

14. The method of any one of claims 10 to 13 of screening for a pharmaceutical composition, wherein the leukemia is MLL or MOZ leukemia.

15. A method of testing for leukemia, which comprises the step of determining the expression level of an M-CSF receptor.

16. An agent for diagnosing leukemia, which is used in the test method of claim 15, and which comprises an oligonucleotide with a length of at least 15 nucleotides and which hybridizes to a region of the M-CSF receptor gene.

17. The agent for diagnosing leukemia, which is used in the test method of claim 15, and which comprises an antibody that recognizes an M-CSF receptor.
